# EUROPEAN PATENT APPLICATION

(11) **EP 2 942 393 A1**
(43) Date of publication of application: **11.11.2015**
(21) Application number: 15157254.2
(22) Date of filing: 07.09.1999
(51) Int. Cl.: C12N 7/02, C12N 15/86

(54) **METHODS FOR GENERATING HIGH TITER HELPER-FREE PREPARATIONS OF RELEASED RECOMBINANT AAV VECTORS**

(30) Priority: 04.09.1998 US 142474; 10.03.1999 US 123685 P
(62) Divisional of application: 07024137.7
(71) Applicant: Genzyme Corporation, Cambridge, MA 02142 (US)
(72) Inventor: Atkinson, Edward Morrow, Seattle, WA 98116 (US); Fung, Victor P., Seattle, WA 98103 (US); Wilkins, Perry C., Bothell, WA 98021 (US); Takeya, Ryan K., Lynwood, WA 98036 (US); Reynolds, Thomas C., San Jose, CA 95138 (US)
(74) Representative: Rai, Monika

(57) **Abstract**

The invention described herein provides methods of producing rAAV particles by culturing producer cells under conditions, such as temperature and pH, that promote release of virus.

## Description

### STATEMENT OF RIGHTS TO INVENTIONS MADE UNDER

### FEDERALLY SPONSORED RESEARCH

This invention was made in part during work supported by a grant from the National Institutes of Health (NIH) R44DK4460. The government may have certain rights in this invention.

### TECHNICAL FIELD

The present invention relates generally to the field of recombinant adeno-associated virus (AAV) vectors and preparations thereof that can be used for gene transfer. More specifically, it relates to methods for generating high titer preparations of recombinant AAV vectors that are substantially free of helper virus (e.g. adenovirus) as well as cellular proteins by release of viral particles from producer cells without cell lysis.

### BACKGROUND ART

Adeno-associated viruses (AAV) have unique features that make them attractive as vectors for gene therapy. Adeno-associated viruses infect a wide range of cell types. However, they are non-transforming, and are not implicated in the etiology of any human disease. Introduction of DNA to recipient host cells generally leads to long-term persistence and expression of the DNA without disturbing the normal metabolism of the cell.

There are at least three desirable features of a recombinant AAV vector preparation for use in gene transfer, especially in human gene therapy. First, it is preferred that the vector should be generated at titers sufficiently high to transduce an effective proportion of cells in the target tissue. Gene therapy *in vivo* typically requires a high number of vector particles. For example, some treatments may require in excess of 10⁸ particles, and treatment of cystic fibrosis by direct delivery to the airway may require in excess of 10¹⁰ particles. Second, it is preferred that the vector preparations should be essentially free of replication-competent AAV (i.e. phenotypically wild-type AAV which can be replicated in the presence of helper virus or helper virus functions). Third, it is preferred that the rAAV vector preparation as a whole be essentially free of other viruses (such as a helper virus used in AAV production) as well as helper virus and cellular proteins, and other components such as lipids and carbohydrates, so as to minimize or eliminate any risk of generating an immune response in the context of gene therapy. This latter point is especially significant in the context of AAV because AAV is a "helper-dependent" virus that requires co-infection with a helper virus (typically adenovirus) or other provision of helper virus functions in order to be effectively replicated and packaged during the process of AAV production; and, moreover, adenovirus has been observed to generate a host immune response in the context of gene therapy applications (see, e.g., Byrnes et al., Neuroscience 66:1015, 1995; McCoy et al., Human Gene Therapy 6:1553, 1995; and Barr et al., Gene Therapy 2:151, 1995). The methods of the present invention address these and other desirable features of rAAV vector preparations, as described and illustrated in detail below.

General reviews of AAV virology and genetics are available elsewhere. The reader may refer *inter alia* to Carter, "Handbook of Parvoviruses", Vol. I, pp. 169-228 (1989), and Berns, "Virology", pp. 1743-1764, Raven Press, (1990). AAV is a replication-defective virus, which means that it relies on a helper virus in order to complete its replication and packaging cycle in a host cell. Helper viruses capable of supporting AAV replication are exemplified by adenovirus, but include other viruses such as herpes and pox viruses. The AAV genome generally comprises the packaging genes *rep* and *cap,* with other necessary functions being provided *in trans* from the helper virus and the host cell.

By way of illustration, the linear genome of serotype AAV2 is terminated at either end by an inverted terminal repeat (ITR) sequence. Between the ITRs are three transcription promoters p5, p19, and p40 that are used to express the *rep* and *cap* genes (Laughlin et al., 1979, Proc. Natl. Acad. Sci. USA, 76:5567-5571). ITR sequences are required in *cis* and are sufficient to provide a functional origin of replication, integration into the cell genome, and efficient excision and rescue from host cell chromosomes or recombinant plasmids. The *rep* and *cap* gene products provide functions for replication and encapsidation of viral genome, respectively, and it is sufficient for them to be present in *trans.*

AAV genomes have been introduced into bacterial plasmids by procedures such as GC tailing (Samulski et al., 1982, Proc. Natl. Acad. Sci. USA, 79:2077-2081), addition of synthetic linkers containing restriction endonuclease cleavage sites (Laughlin et al., 1983, Gene, 23:65-73) or by direct, blunt-end ligation (Senapathy & Carter, 1984, J. Biol. Chem., 259:4661-4666). Transfection of such AAV recombinant plasmids into mammalian cells with an appropriate helper virus results in rescue and excision of the AAV genome free of any plasmid sequence, replication of the rescued genome and generation of progeny infectious AAV particles.

Recombinant AAV vectors comprising a heterologous polynucleotide of therapeutic interest may be constructed by substituting portions of the AAV coding sequence in bacterial plasmids with the heterologous polynucleotide. General principles of rAAV vector construction are also reviewed elsewhere. See, e.g., Carter, 1992, Current Opinions in Biotechnology, 3:533-539; and Muzyczka, 1992, Curr. Topics in Microbiol. and Immunol., 158:97-129). The AAV ITRs are generally retained, since packaging of the vector requires that they be present in *cis.* However, other elements of the AAV genome, in particular, one or more of the packaging genes, may be omitted. The vector plasmid can be packaged into an AAV particle by supplying the omitted packaging genes in *trans* via an alternative source. A number of publications describe various approaches to producing rAAV vectors. Ratschin et al., Mol. Cell. Biol. 4:2072 (1984); Hermonat et al., Proc. Natl. Acad. Sci. USA, 81:6466 (1984); Tratschin et al., Mol. Cell. Biol. 5:3251 (1985); McLaughlin et al., J. Virol., 62:1963 (1988); Lebkowski et al., 1988 Mol. Cell. Biol., 7:349 (1988). Samulski et al., J. Virol., 63:3822-3828 (1989); Flotte et al., Am. J. Respir. Cell. Mol. Biol. 7:349 (1992); U.S. Patent 5,173,414; WO 95/13365 (Targeted Genetics Corporation and Johns Hopkins University) and corresponding U.S. Patent No. 5,658,776 (by Flotte et al.); WO 95/13392 (Trempe et al.); WO 96/17947 (by Targeted Genetics Corporation, J. Allen).

Since high titers of rAAV vector preparations are particularly useful, but the production of high titers of rAAV, particularly in large-scale procedures, can lead to the generation of significant quantities of contaminating helper virus (e.g. adenovirus or "Ad"), helper virus proteins (e.g. Ad proteins), and/or cellular proteins, it became especially important to design scalable methods for the production of rAAV that can be used for the generation of high-titer preparations that are substantially free of contaminating virus and/or viral or cellular proteins.

Methods for generating high-titer preparations of recombinant AAV vectors have been described. International Patent Application No. PCT/US98/18600 describes culturing a cell line which can produce rAAV vector upon infection with a helper virus; infecting the cells with a helper virus, such as adenovirus; and lysing the cells. AAV and other viral production methods and systems are also described in, for example, WO 97/09441 (PCT/US96/14423); WO 97/08298 (PCT/US96/13872); WO 97/21825 (PCT/US96/20777); WO 97/06243 (PCT/FR96/01064); WO 99/11764; Perrin et al. (1995) Vaccine 13:1244-1250; Paul et al. (1993) Human Gene Therapy 4:609-615; Clark et al. (1996) Gene Therapy 3:1124-1132.

Prior art methods used to produce recombinant AAV particle using packaging cells required a cell lysis step due to the pervasive belief that AAV is not released from producer cells in any appreciable amount without lysing the cells. See, for example, Chirico and Trempe (1998), J. Viral. Methods 76:31-41. However, the cell lysate contains various cellular components which must be separated from the rAAV vector before it is suitable for *in vivo* use.

The present disclosure provides methods for achieving high-titer production of rAAV vectors released from a producer cell without lysing the cell(s), and demonstrates that such techniques can be employed for the large-scale production of recombinant AAV vector preparations.

All publications and patent applications cited herein are hereby incorporated by reference in their entirety.

### SUMMARY OF THE INVENTION

This invention provides methods and materials for generating high titer preparations of adeno-associated virus (AAV) that are substantially free of helper virus, helper virus proteins, and cellular proteins and other components. The methods entail release of rAAV particle from producer cells without actively lysing the cells as is typical in the art, which provides a distinct and significant advantage over previously described production methods. The methods are also applicable to viruses which are non-lytic and/or generally not released (i.e., non-budding viruses).

Accordingly, in one aspect, the invention provides methods of generating a population of virus particles, such as recombinant adeno-associated virus (rAAV) particles. comprising the step of: a) incubating a producer cell in a cell culture medium under conditions which promote release of AAV particles from the cell, whereby rAAV particles are released from the producer cell into the culture medium, and wherein the producer cell comprises (i) one or more AAV packaging genes, wherein each said AAV packaging gene encodes an AAV replication or encapsidation protein; (ii) a recombinant AAV (rAAV) vector that comprises a heterologous non-AAV polynucleotide flanked by at least one AAV inverted terminal repeat (ITR); and (iii) a helper virus for AAV or helper virus function for AAV. The released rAAV particles may then be collected, or harvested, from the culture medium. Conditions which promote release of rAAV viral particles are described herein and include, but are not limited to, pH, osmolality, dissolved oxygen, enriched media, and temperature.

In some embodiments, the methods further include various purification and/or inactivation steps. In some of these embodiments, the method further comprises the steps of: chromatographing the AAV producer cell supernatant on a plurality of ion-exchange resins comprising at least one positively-charged anion exchange resin and at least one negatively-charged cationic exchange resin to generate a purified population of rAAV vector particles, or chromatographing the AAV producer cell supernatant on an anion exchange resin followed by tangential flow filtration (TFF). Heparin sulphate chromotography can also be used to further purify the virus.

In the methods of the invention, cell culture can be carried out such that the cells are in suspension or under conditions that promote adherence of cells to a solid support. Accordingly, in some embodiments, the producer cell is cultured in a vessel selected from the group consisting of a tissue culture flask, a roller bottle, a spinner flask, a tank reactor, a fermentor, and a bioreactor, a flat stock reactor, a hollow fiber system, a packed bed reactor and optionally using a microcarrier.

In some embodiments of the invention, recombinant AAV vector preparations produced by the methods result in a purified population of rAAV vector particles which is substantially free of replication-competent AAV and of helper virus and cellular proteins as well as substantially free cellular DNA.

The present invention further provides a population of rAAV particles, produced according to any of the production methods of this invention. Preferably, the population of particles contains no more than about one infectious adenovirus particles per thousand infectious rAAV particles, preferably less than one per 10⁶ rAAV, still more preferably less than about one in 10⁹, even more preferably less than about one in 10¹⁰.

These and other embodiments of the invention are outlined in the description that follows.

### BRIEF DESCRIPTION OF THE DRAWINGS

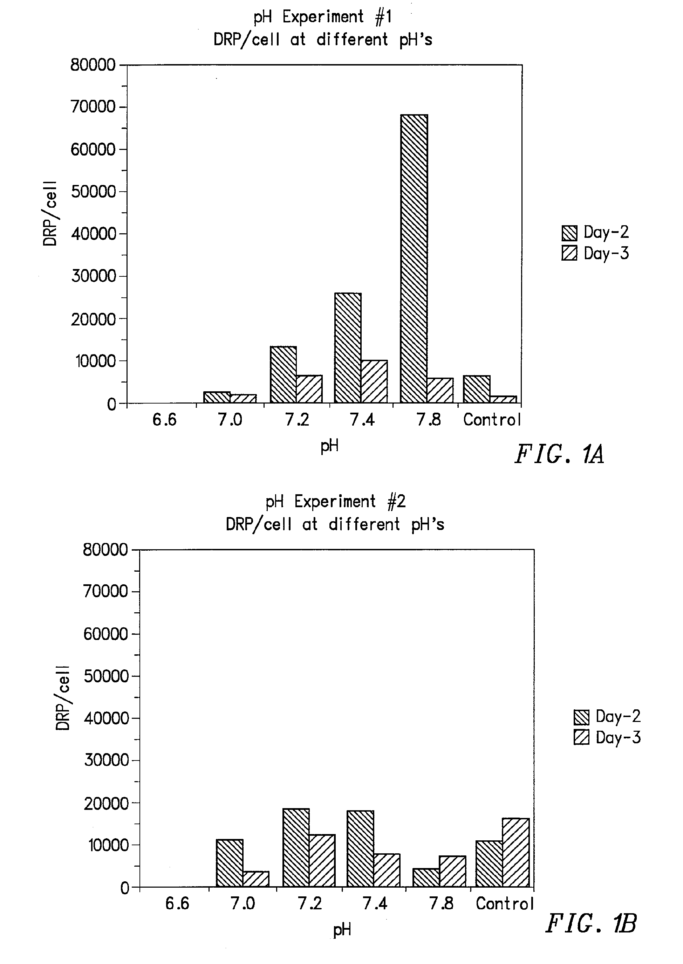

Figures 1A and 1B are bar graphs depicting the results of two separate experiments, expressed as (DNase resistant particles (DRP) per cell at the various pH levels. Cell cultures were maintained at the indicated pH levels, and cell lysates were assayed at day 2 (solid bars) and day 3 (hatched bars) post-infection.

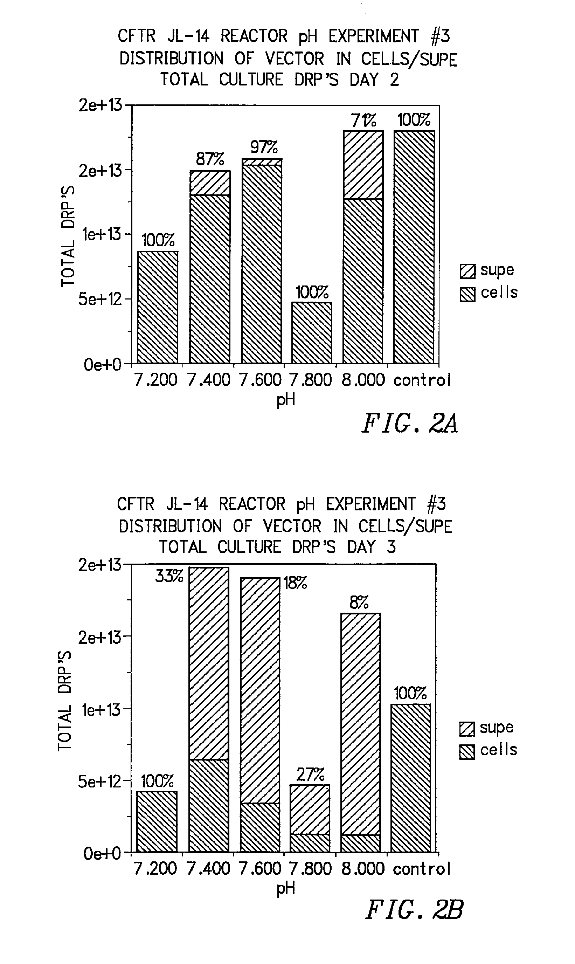

Figures 2A and 2B are bar graphs and depict the results, expressed as total DRPs, of rAAV production, at day 2 (2A) and day 3 (2B) post-infection, in bioreactors maintained at various pH levels. Percentages above each bar are percentages of total DRPs in the cell lysate. The solid portion of each bar represents DRPs in cell lysates, while the hatched portion of each bar represents the DRPs in the cell culture medium. Percentages above each bar indicate the percentage of total DRPs in the cell lysate.

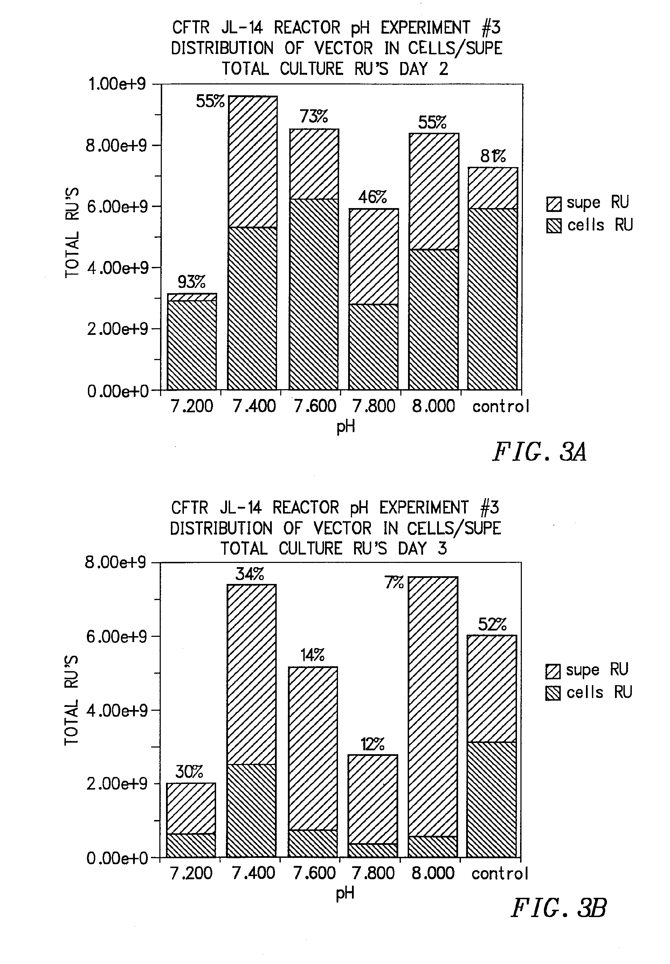

Figures 3A and 3B are bar graphs depicting the total replication units (RU), at day 2 (3A) and day 3 (3B) post-infection, in the culture media (hatched portion of each bar) and cell lysates (solid portion of each bar) when cultures were maintained at the indicated pH levels. Percentages above each bar indicate the percentage of total RUs in the cell lysate.

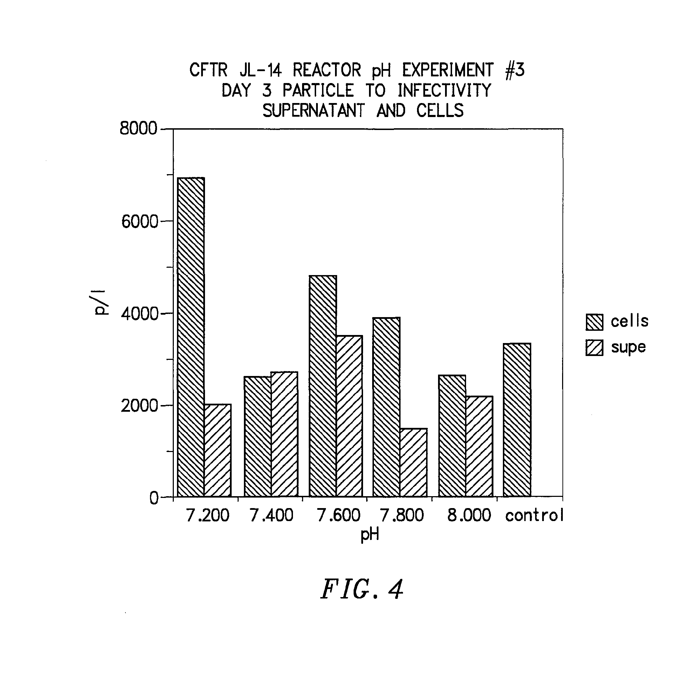

Figure 4 is a bar graph depicting the particle:infectivity (P/I) ratio of rAAV particles harvested from cell lysates (solid portion of each bar) and cell culture medium (hatched portion of each bar) at day 3 post-infection from bioreactors maintained at the indicated pH levels.

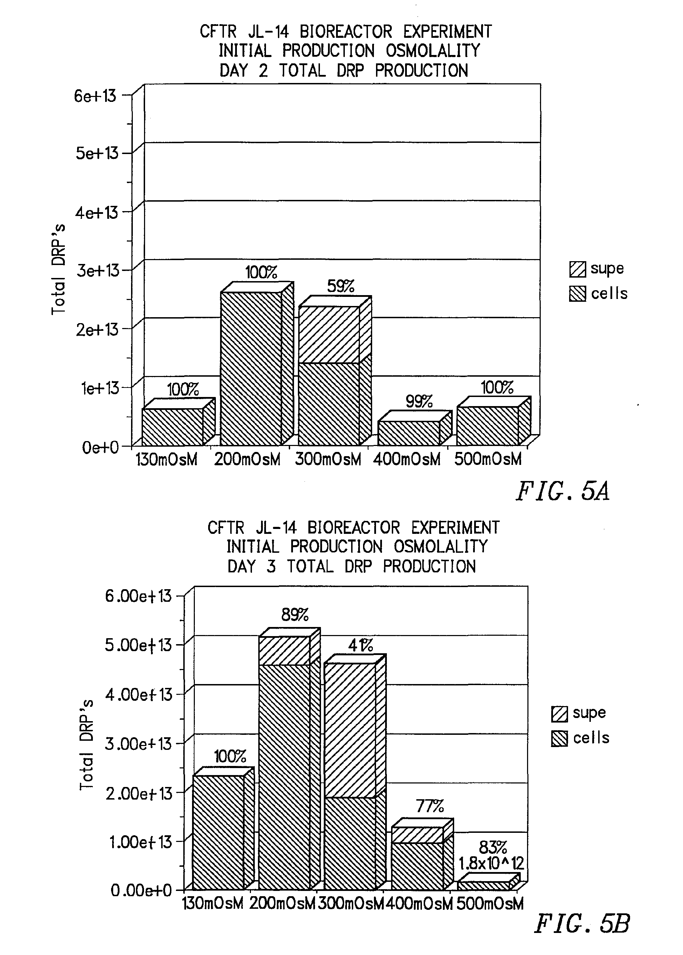

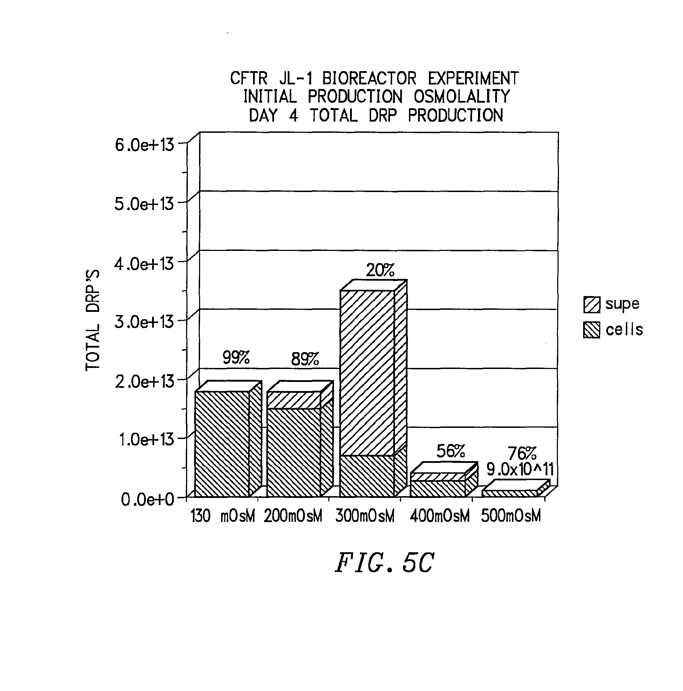

Figures 5A, 5B, and 5C are bar graphs depicting the total DRPs in cell lysates (solid portion of each bar) and cell culture media (hatched portion of each bar) on day 2 (5A), day 3 (5B), and day 4 (5C) post-infection in bioreactors in which the cell culture media contained the indicated starting osmolality. Percentages above each bar indicate the percentage of total DRPs in the cell lysate.

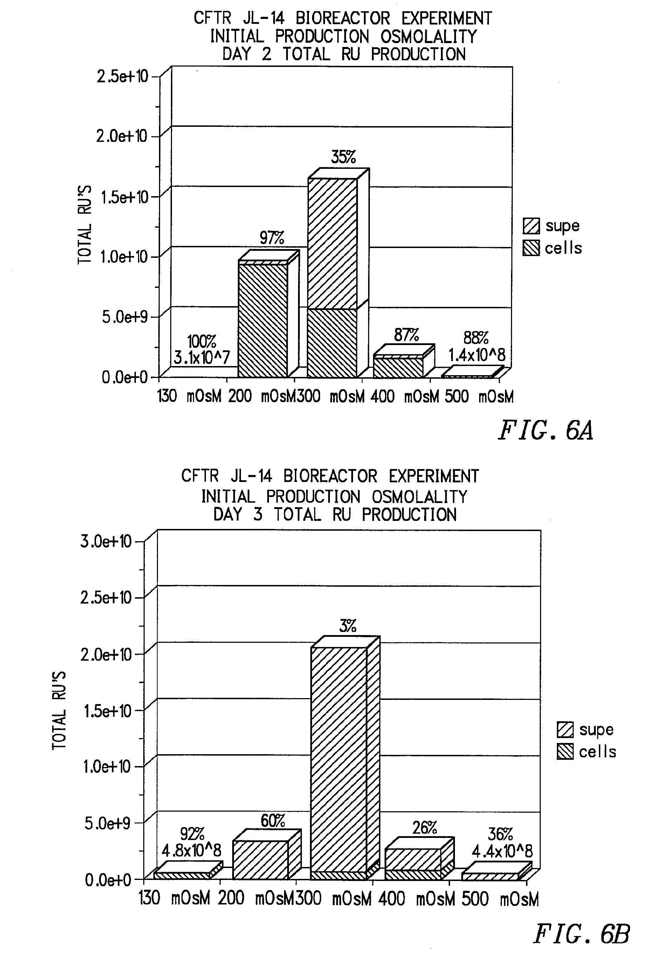

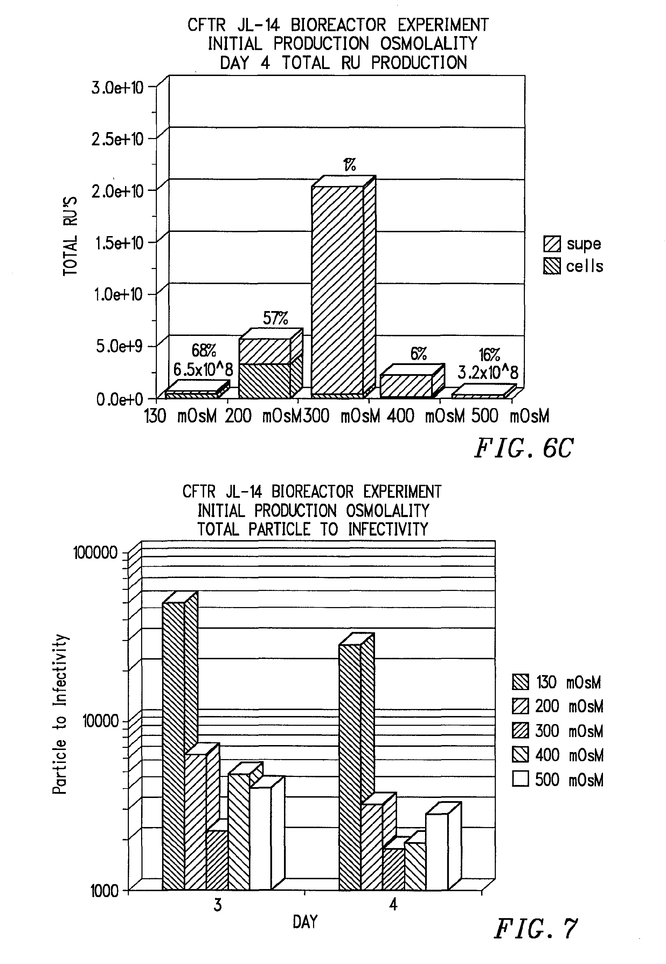

Figures 6A, 6B, and 6C are bar graphs depicting the total RUs in cell lysates (solid portion of each bar) and cell culture media (hatched portion of each bar) on day 2 (6A), day 3 (6B), and day 4 (6C) post-infection in bioreactors in which the cell culture media contained the indicated starting osmolality. Percentages above each bar indicate the percentage of total RUs in the cell lysates.
Figure 7 is a bar graph depicting the P/I ratio of rAAV particles in cell culture media at days 3 and 4 from bioreactor cultures with the indicated starting osmolalities.

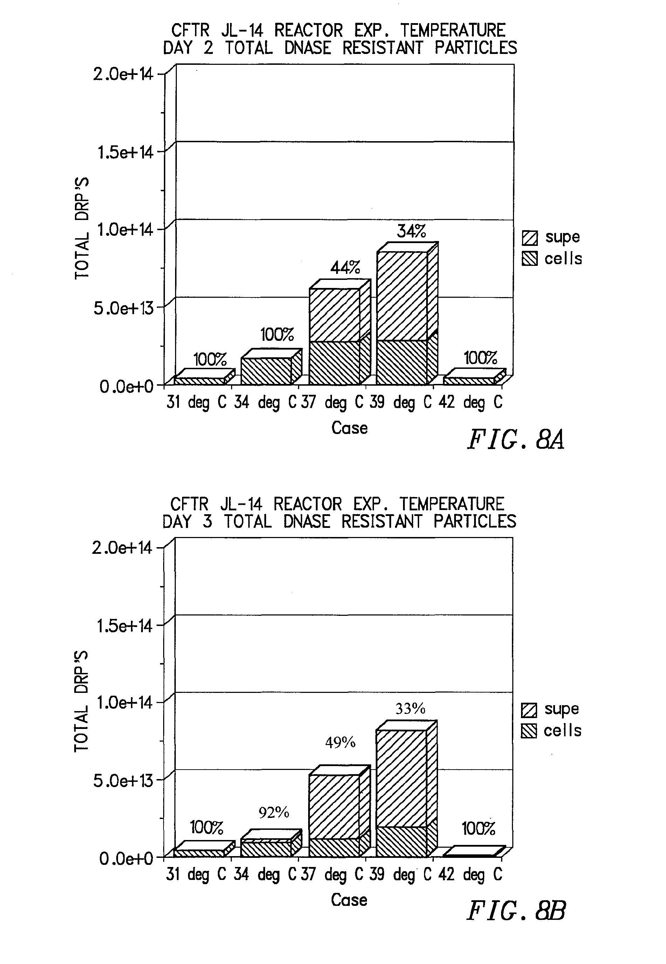

Figures 8A-C are bar graphs depicting the total DRPs in cell lysates (solid portion of each bar) and cell culture media (hatched portion of each bar) on day 2 (8A), day 3 (8B), and day 4 (8C) post-infection in bioreactors in which the cell culture media was maintained at the indicated temperature. Percentages above each bar indicate the percentage of total DRPs in the cell lysate.

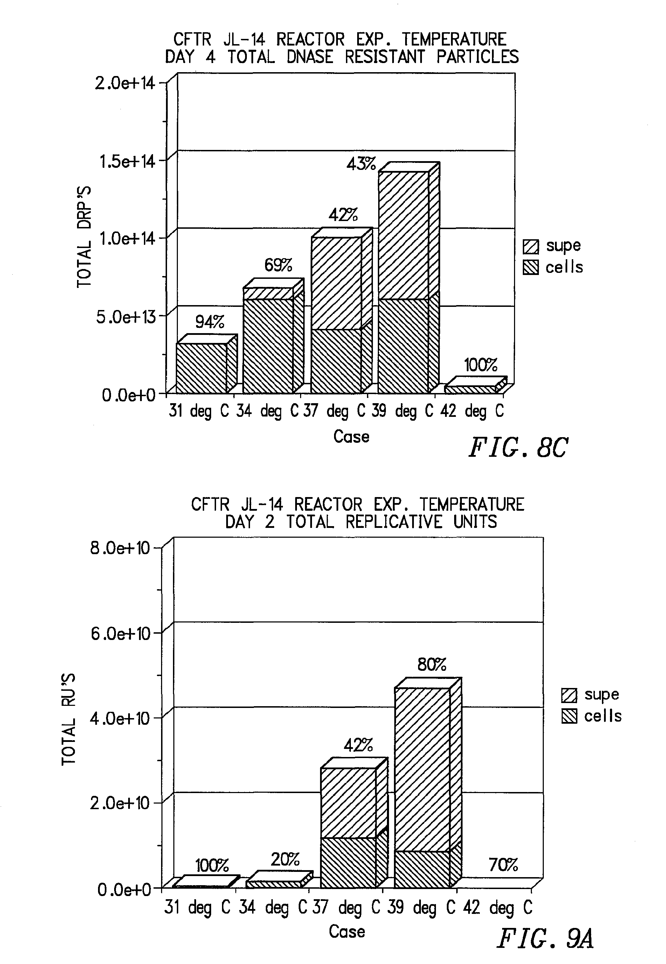

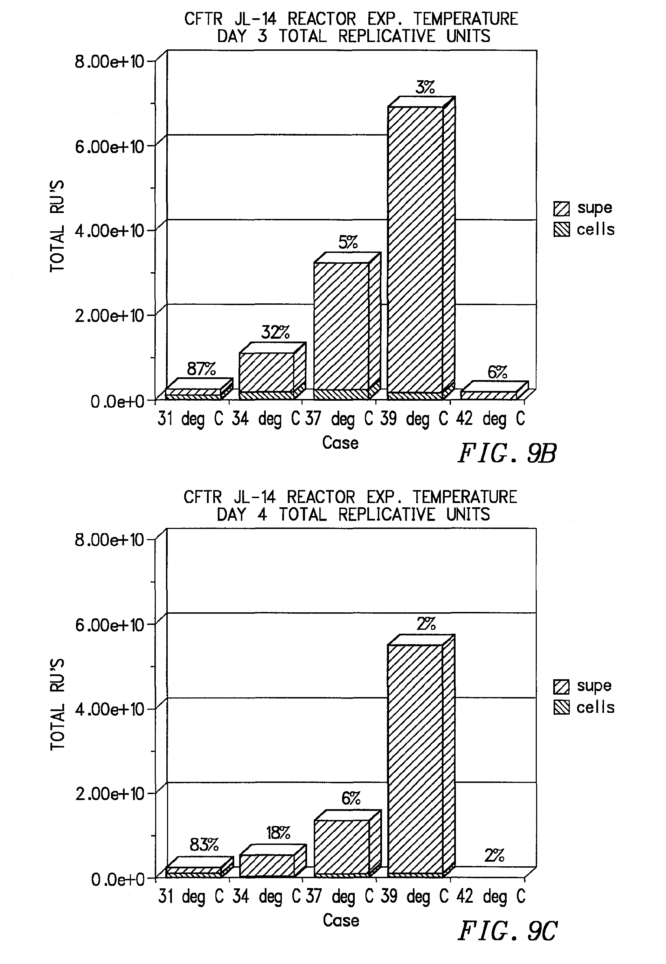

Figures 9A, 9B, and 9C are bar graphs depicting the total RUs in cell lysates (solid portion of each bar) and cell culture media (hatched portion of each bar) on day 2 (9A), day 3 (9B), and day 4 (9C) post-infection in bioreactors in which the cell culture media was maintained at the indicated temperature. Percentages above each bar in Figure 9A indicate the percentage of total RUs in the cell culture medium. Percentages above each bar in Figure 9A indicate the percentage of total RUs in the cell lysate.

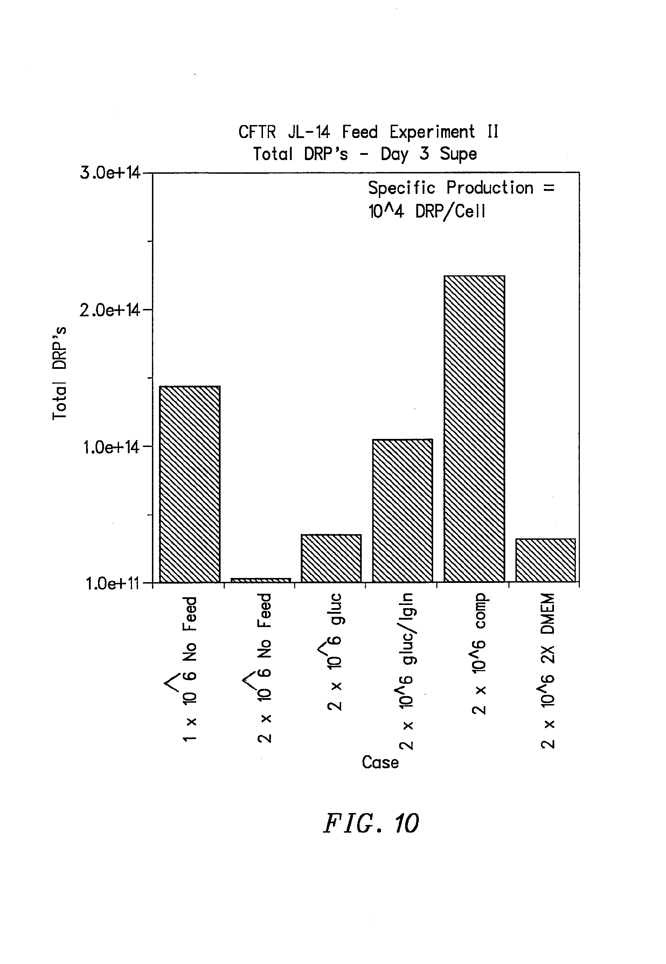

Figure 10 is a bar graph depicting the total DRPs in the culture media three days post-infection in cultures grown in the various media indicated.

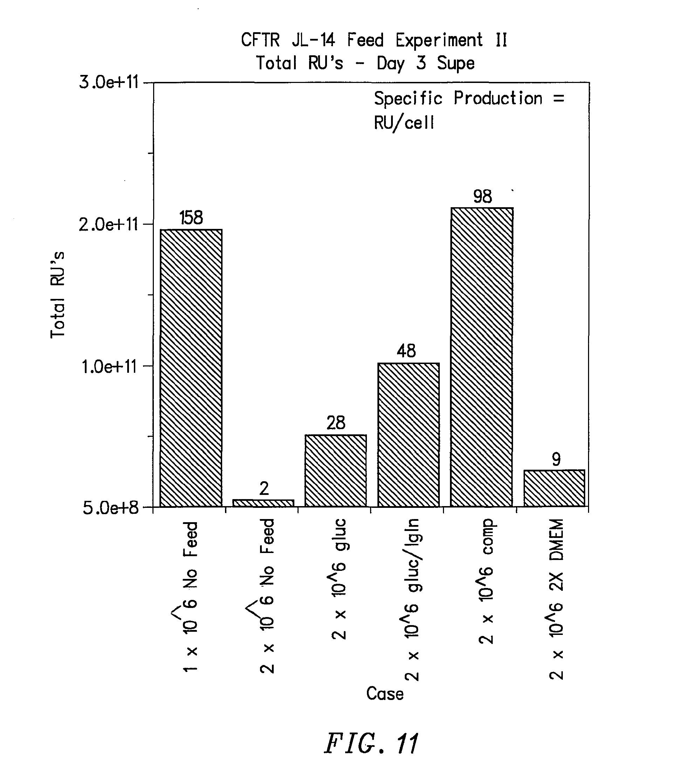

Figure 11 is a bar graph depicting the RUs in the culture media three days post-infection in cultures grown in the various media indicated.

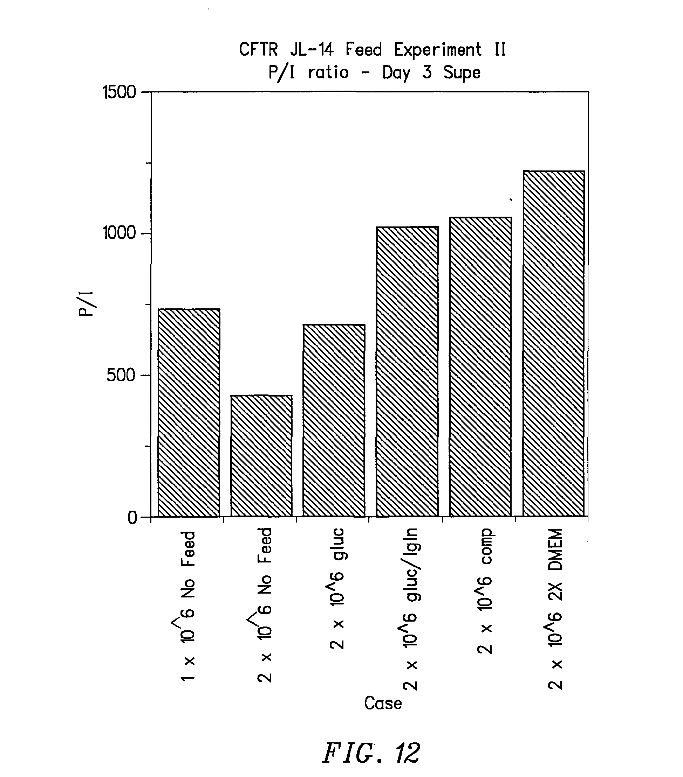

Figure 12 is a bar graph depicting the P/I ratio of viral particles in the cell culture media when cultures were grown in the various media indicated.

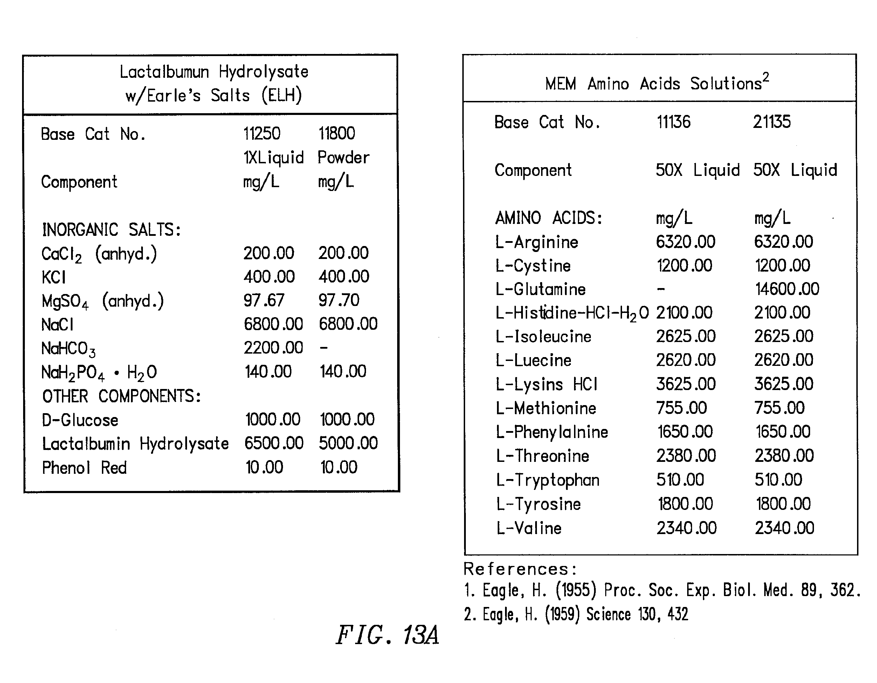

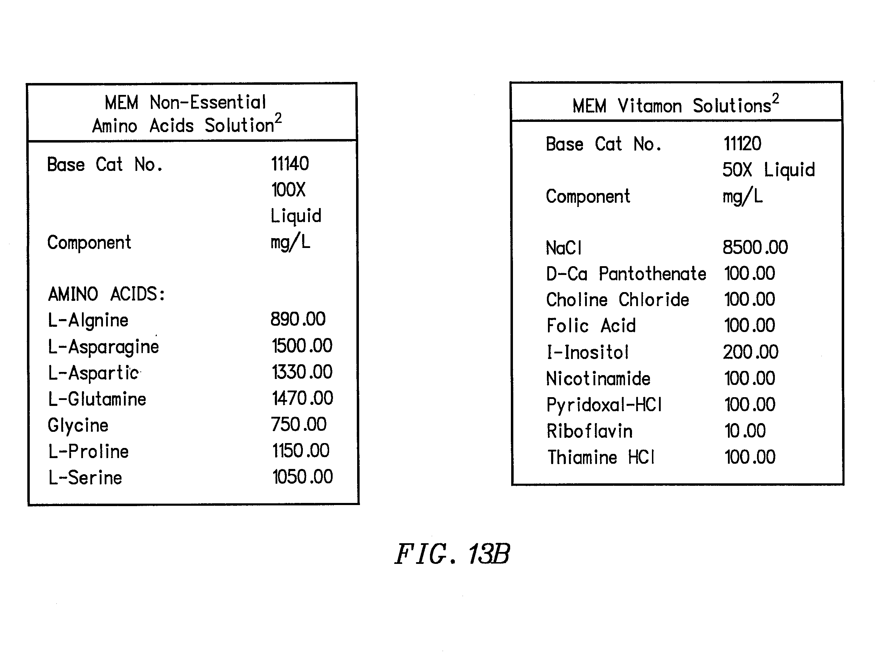

Figure 13 provides amino acid and vitamin compositions for the media supplements described in Example 6.

### MODES FOR CARRYING OUT THE INVENTION

We have discovered methods for generating high titer preparations of adeno-associated virus (AAV) that are substantially free of helper virus, helper virus proteins, and cellular proteins and other components. The methods generally entail culturing (which generally involves maintaining) producer cells under conditions which promote release of rAAV particles from the producer cells.

It is well-established in the AAV field that AAV is not released from the cell unless the cell is lysed, but remains in the nucleus of the cell. Accordingly, the pervasive and universal belief is that, in order to produce rAAV particles, the cells must be lysed. In contrast to the teachings of the field, we have discovered that rAAV particles can be released from cells without lysing the cells, and further that release of rAAV particles can be increased by maintaining the producer cells under various controlled environmental conditions. Using these conditions, rAAV particles can be produced at titers higher than previously obtained. Cells cultured under the conditions described herein produce more virus per cell and release more virus into the culture medium, and, even more significantly, may release a population of AAV with higher infectivity than AAV which is retained within the cell. In other words, the DNAse resistant particle to infectivity ratio can be smaller in the AAV population released into the cell culture medium compared to this ratio of AAV retained within the cell (see, e.g., Figure 4). Furthermore, since lysis is not an obligatory step in the methods of the present invention, the rAAV particles can be collected from the cell supernatant, thus simplifying subsequent optional purification steps. Alternatively, lysis could also be performed.

In some embodiments, the invention provides methods of release, or preferential release, of infectious viral particles. This preferential release of infectious particles is particularly significant in the viral production context, in which it is highly desirable to produce a population containing a large number of infectious particles as opposed to noninfective particles.

It is understood that the methods and principles described herein are applicable to a number of other viruses which are normally retained (i.e., not released), particularly adenovirus. AAV is exemplified herein.

Various methods for the generation and processing of AAV particles in mammalian cells are described in detail below, and illustrations of the use of such techniques are provided in the Examples following. The rAAV particles produced by the methods of this invention are particularly useful as gene transfer vectors. Methods of using such vectors are known in the art and need not be described herein.

By way of introduction, it is typical to employ a host or "producer" cell for rAAV vector replication and packaging. Such a producer cell (usually a mammalian host cell) generally comprises or is modified to comprise several different types of components for rAAV production. The first component is a recombinant adeno-associated viral (rAAV) vector genome (or "rAAV pro-vector") that can be replicated and packaged into vector particles by the host packaging cell. The rAAV pro-vector will normally comprise a heterologous polynucleotide (or "transgene"), with which it is desired to genetically alter another cell in the context of gene therapy (since the packaging of such a transgene into rAAV vector particles can be effectively used to deliver the transgene to a variety of mammalian cells). The transgene is preferably flanked by two AAV inverted terminal repeats (ITRs) which comprise sequences that are recognized during excision, replication and packaging of the AAV vector, as well as during integration of the vector into a host cell genome. A second component is a helper virus that can provide helper functions for AAV replication. Although adenovirus is commonly employed, other helper viruses can also be used as is known in the art. Alternatively, the requisite helper virus functions can be isolated genetically from a helper virus and the encoding genes can be used to provide helper virus functions *in trans.* The AAV vector elements and the helper virus (or helper virus functions) can be introduced into the host cell either simultaneously or sequentially in any order. The final components for AAV production to be provided in the producer cell are "AAV packaging genes" such as AAV *rep* and *cap* genes that provide replication and encapsidation proteins, respectively. Several different versions of AAV packaging genes can be provided (including wild-type *rep-cap* cassettes as well as modified *rep* and/or *cap* cassettes in which the *rep* and/or *cap* genes can be left under the control of the native promoters or operably linked to heterologous promoters). Such AAV packaging genes can be introduced either transiently or stably into the host packaging cell, as is known in the art and described in more detail below.

Following the methods of the invention, rAAV particles are released into the cell culture medium ("supernatant") from intact (i.e., not lysed) cells. After culturing the host cells under conditions that permit AAV replication, encapsidation, and release the supernatant can be processed to generate high titer preparations of adeno-associated virus (AAV) that are substantially free of helper virus, helper virus proteins, cellular proteins, and, significantly, cellular DNA. Detailed descriptions of processing techniques and illustrative protocols employing such techniques are provided below.

### Definitions

A "vector" as used herein refers to a macromolecule or association of macromolecules that comprises or associates with a polynucleotide and which can be used to mediate delivery of the polynucleotide to a cell. Illustrative vectors include, for example, plasmids, viral vectors, liposomes and other gene delivery vehicles.

"AAV" is an abbreviation for adeno-associated virus, and may be used to refer to the virus itself or derivatives thereof. The term covers all subtypes and both naturally occurring and recombinant forms, except where required otherwise. The abbreviation "rAAV" refers to recombinant adeno-associated virus, also referred to as a recombinant AAV vector (or "rAAV vector").

An "rAAV vector" as used herein refers to an AAV vector comprising a polynucleotide sequence not of AAV origin (i.e., a polynucleotide heterologous to AAV), typically a sequence of interest for the genetic transformation of a cell. In preferred vector constructs of this invention, the heterologous polynucleotide is flanked by at least one, preferably two AAV inverted terminal repeat sequences (ITRs). The term rAAV vector encompasses both rAAV vector particles and rAAV vector plasmids.

An "rAAV vector particle" or "rAAV particle" refers to a viral particle composed of at least one AAV capsid protein (preferably by all of the capsid proteins of a wild-type AAV) and an encapsidated rAAV vector. Thus, production of rAAV particle necessarily includes production of rAAV vector, as such a vector is contained within an rAAV particle.

"Packaging" refers to a series of intracellular events that result in the assembly and encapsidation of an AAV particle.

AAV *"rep"* and *"cap"* genes refer to polynucleotide sequences encoding replication and encapsidation proteins of adeno-associated virus. They have been found in all AAV serotypes examined, and are described below and in the art. AAV *rep* and *cap* are referred to herein as AAV "packaging genes".

A "helper virus" for AAV refers to a virus that allows AAV (e.g. wild-type AAV) to be replicated and packaged by a mammalian cell. A variety of such helper viruses for AAV are known in the art, including adenoviruses, herpesviruses and poxviruses such as vaccinia. The adenoviruses encompass a number of different subgroups, although Adenovirus type 5 of subgroup C is most commonly used. Numerous adenoviruses of human, non-human mammalian and avian origin are known and available from depositories such as the ATCC. Viruses of the herpes family include, for example, herpes simplex viruses (HSV) and Epstein-Barr viruses (EBV), as well as cytomegaloviruses (CMV) and pseudorabies viruses (PRV); which are also available from depositories such as ATCC.

"Helper virus function(s)" refers to function(s) encoded in a helper virus genome which allow AAV replication and packaging (in conjunction with other requirements for replication and packaging described herein). As described herein, "helper virus function" may be provided in a number of ways, including by providing helper virus or providing, for example, polynucleotide sequences encoding the requiste function(s) to a producer cell in trans.

The term "tsHV" refers to a temperature-sensitive helper virus, which can provide helper functions for AAV replication and packaging but is temperature-sensitive with respect to its own replication (i.e. it can replicate at a "permissive" temperature but replicates at lower efficiency, or preferably not at all, at a "non-permissive" temperature). The ability of the tsHV to provide help for AAV replication may also be temperature sensitive, but preferred tsHV for use with this invention efficiently support AAV replication at temperatures at which AAV can replicate but which are non-permissive for replication of the tsHV. Examples of such tsHV are described below.

An "infectious" virus or viral particle is one that comprises a polynucleotide component which it is capable of delivering into a cell for which the viral species is trophic. The term does not necessarily imply any replication capacity of the virus. Assays for counting infectious viral particles are described elsewhere in this disclosure and in the art. Viral infectivity can be expressed as the P:I ratio, or the ratio of total viral particles to infective viral particles.

A "replication-competent" virus (e.g. a replication-competent AAV) refers to a phenotypically wild-type virus that is infectious, and is also capable of being replicated in an infected cell (i.e. in the presence of a helper virus or helper virus functions). In the case of AAV, replication competence generally requires the presence of functional AAV packaging genes. Preferred rAAV vectors as described herein are replication-incompetent in mammalian cells (especially in human cells) by virtue of the lack of one or more AAV packaging genes. Preferably, such rAAV vectors lack any AAV packaging gene sequences in order to minimize the possibility that replication competent AAV are generated by recombination between AAV packaging genes and an incoming rAAV vector. Preferred rAAV vector preparations as described herein are those which contain few if any replication competent AAV (rcAAV) (preferably less than about 1 rcAAV per 10² rAAV particles, more preferably less than about 1 rcAAV per 10⁴ rAAV particles, still more preferably less than about 1 rcAAV per 10⁸ rAAV particles, even more preferably less than about 1 rcAAV per 10¹² rAAV particles, most preferably no rcAAV).

"Release" of rAAV particles means that rAAV particles enter the cell culture medium from an intact producer cell, i.e., the rAAV particle is released without lysing the cell. It is understood that, in a given producer cell culture, some cells lyse, for example, upon cell death. However, this invention provides methods which promote release of rAAV particle without performing deliberate cell lysis, as it typically done in the art. The terms "release" and "secretion" from a producer cell are used interchangeably herein.

The term "condition that promotes release of rAAV particles" from a producer cell, as used herein, refers to a condition for growing producer cells which lead to increased, or enhanced, rAAV particle release from the producer cell into the culture medium. Conditions which promote release of rAAV from the producer cell into the culture medium are described herein, and are generally, but not necessarily, conditions which enhance cellular metabolism. "Promoting release" of rAAV particles from a producer cell into the culture medium means that the rAAV release from the producer cell is increased when compared to rAAV release from a producer cell not cultured under the environmental condition(s) which enhance release. The increase may be any detectable increase, such as at least about 1%, at least about 5%, at least about 10%, more preferably at least about 20%, more preferably at least about 25%, more preferably at least about 35%, more preferably at least about 50%, more preferably at least about 60%, more preferably at least about 65%, more preferably at least about 75%, more preferably at least about 80%, more preferably at least about 85%, more preferably at least about 90%, more preferably at least about 100% or 2-fold, more preferably at least about 5-fold, more preferably at least about 10-fold, more preferably at least about 20-fold, even more preferably at least about 50-fold. As is well known in the art, when a cell population is grown under a given starting culture condition, the cells' metabolic by-products will change certain of the culture conditions, such as pH and osmolality. Under environmental conditions which promote rAAV particle release, one or more of these parameters is controlled as necessary, i.e, monitored at regular intervals and adjusted to maintain the parameter within a suitable range (i.e., a range that promotes release). Setting and/or control of these conditions will be discussed in more detail below.

The term "polynucleotide" refers to a polymeric form of nucleotides of any length, including deoxyribonucleotides or ribonucleotides, or analogs thereof. A polynucleotide may comprise modified nucleotides, such as methylated nucleotides and nucleotide analogs, and may be interrupted by non-nucleotide components. If present, modifications to the nucleotide structure may be imparted before or after assembly of the polymer. The term polynucleotide, as used herein, refers interchangeably to double- and single-stranded molecules. Unless otherwise specified or required, any embodiment of the invention described herein that is a polynucleotide encompasses both the double-stranded form and each of two complementary single-stranded forms known or predicted to make up the double-stranded form.

A "gene" refers to a polynucleotide containing at least one open reading frame that is capable of encoding a particular protein after being transcribed and translated.

"Recombinant", as applied to a polynucleotide means that the polynucleotide is the product of various combinations of cloning, restriction or ligation steps, and other procedures that result in a construct that is distinct from a polynucleotide found in nature. A recombinant virus is a viral particle comprising a recombinant polynucleotide. The terms respectively include replicates of the original polynucleotide construct and progeny of the original virus construct.

A "control element" or "control sequence" is a nucleotide sequence involved in an interaction of molecules that contributes to the functional regulation of a polynucleotide, including replication, duplication, transcription, splicing, translation, or degradation of the polynucleotide. The regulation may affect the frequency, speed, or specificity of the process, and may be enhancing or inhibitory in nature. Control elements known in the art include, for example, transcriptional regulatory sequences such as promoters and enhancers. A promoter is a DNA region capable under certain conditions of binding RNA polymerase and initiating transcription of a coding region usually located downstream (in the 3' direction) from the promoter.

"Operatively linked" or "operably linked" refers to a juxtaposition of genetic elements, wherein the elements are in a relationship permitting them to operate in the expected manner. For instance, a promoter is operatively linked to a coding region if the promoter helps initiate transcription of the coding sequence. There may be intervening residues between the promoter and coding region so long as this functional relationship is maintained.

An "expression vector" is a vector comprising a region which encodes a polypeptide of interest, and is used for effecting the expression of the protein in an intended target cell. An expression vector also comprises control elements operatively linked to the encoding region to facilitate expression of the protein in the target. The combination of control elements and a gene or genes to which they are operably linked for expression is sometimes referred to as an "expression cassette," a large number of which are known and available in the art or can be readily constructed from components that are available in the art.

"Heterologous" means derived from a genotypically distinct entity from that of the rest of the entity to which it is being compared. For example, a polynucleotide introduced by genetic engineering techniques into a plasmid or vector derived from a different species is a heterologous polynucleotide. A promoter removed from its native coding sequence and operatively linked to a coding sequence with which it is not naturally found linked is a heterologous promoter.

"Genetic alteration" refers to a process wherein a genetic element is introduced into a cell other than by mitosis or meiosis. The element may be heterologous to the cell, or it may be an additional copy or improved version of an element already present in the cell. Genetic alteration may be effected, for example, by transfecting a cell with a recombinant plasmid or other polynucleotide through any process known in the art, such as electroporation, calcium phosphate precipitation, or contacting with a polynucleotide-liposome complex. Genetic alteration may also be effected, for example, by transduction or infection with a DNA or RNA virus or viral vector. Preferably, the genetic element is introduced into a chromosome or mini-chromosome in the cell; but any alteration that changes the phenotype and/or genotype of the cell and its progeny is included in this term.

A cell is said to be "stably" altered, transduced, or transformed with a genetic sequence if the sequence is available to perform its function during extended culture of the cell in vitro. In preferred examples, such a cell is "inheritably" altered in that a genetic alteration is introduced which is also inheritable by progeny of the altered cell.

The terms "polypeptide", "peptide" and "protein" are used interchangeably herein to refer to polymers of amino acids of any length. The terms also encompass an amino acid polymer that has been modified; for example, disulfide bond formation, glycosylation, lipidation, or conjugation with a labeling component. Polypeptides such as "CFTR", "p53", "E1A" and the like, when discussed in the context of gene therapy and compositions therefor, refer to the respective intact polypeptide, or any fragment or genetically engineered derivative thereof, that retains the desired biochemical function of the intact protein. Similarly, references to CFTR, p53, E1A genes, and other such genes for use in gene therapy (typically referred to as "transgenes" to be delivered to a recipient cell), include polynucleotides encoding the intact polypeptide or any fragment or genetically engineered derivative possessing the desired biochemical function.

An "isolated" plasmid, virus, or other substance refers to a preparation of the substance devoid of at least some of the other components that may also be present where the substance or a similar substance naturally occurs or is initially prepared from. Thus, for example, an isolated substance may be prepared by using a purification technique to enrich it from a source mixture. Enrichment can be measured on an absolute basis, such as weight per volume of solution, or it can be measured in relation to a second, potentially interfering substance present in the source mixture. Increasing enrichments of the embodiments of this invention are increasingly more preferred. Thus, for example, a 2-fold enrichment is preferred, 10-fold enrichment is more preferred, 100-fold enrichment is more preferred, 1000-fold enrichment is even more preferred.

A preparation of AAV is said to be "substantially free" of helper virus if the ratio of infectious AAV particles to infectious helper virus particles is at least about 10²:1; preferably at least about 10⁴:1, more preferably at least about 10⁶:1; still more preferably at least about 10⁸:1. Preparations are also preferably free of equivalent amounts of helper virus proteins (i.e. proteins as would be present as a result of such a level of helper virus if the helper virus particle impurities noted above were present in disrupted form). Viral and/or cellular protein contamination can generally be observed as the presence of Coomassie staining bands or silver stained bands on SDS gels (e.g. the appearance of bands other than those corresponding to the AAV capsid proteins VP1, VP2 and VP3).

"Efficiency" when used in describing viral production, replication or packaging refers to useful properties of the method; in particular, the growth rate and the number of virus particles produced per cell. "High efficiency" production indicates production of at least 100 viral particles per cell; preferably at least about 10,000 and more preferably at least about 100,000 particles per cell, over the course of the culture period specified. Even more preferably, "high efficiency" production encompasses these production levels of particles per cell as well as the maximum number of cells producing particles, such as at least about 10%, preferably at least about 20%, preferably at least 30%, preferably at least 50%, preferably at least 75%. Example 6 describes culture conditions ("complete" medium) which resulted in 93,000 - 123,000 rAAV particles per producer cell. In the context of the present invention, efficiency may also be considered in terms of percentage, or extent, of release of viral particles compared to viral particles retained in the cell. Efficiency may also be considered in terms of ratio or relative proportion of total viral particles to infectious viral particles (such as a "P/I" ratio). Assays for determining parameters of efficiency of production, such as replicative units and infectious center assay, are known in the art.

### General techniques

The practice of the present invention will employ, unless otherwise indicated, conventional techniques of molecular biology, virology, animal cell culture and biochemistry which are within the skill of the art. Such techniques are explained fully in the literature. See, for example, "Molecular Cloning: A Laboratory Manual", Second Edition (Sambrook, Fritsch & Maniatis, 1989); "Animal Cell Culture" (R.I. Freshney, ed., 1987); "Gene Transfer Vectors for Mammalian Cells" (J.M. Miller & M.P. Calos, eds., 1987); "Current Protocols in Molecular Biology" (F.M. Ausubel et al., eds., 1987); "Current Protocols in Protein Science" (John E Coligan, et al. eds. Wiley and Sons, 1995); and "Protein Purification: Principles and Practice" (Robert K. Scopes, Springer-Verlag, 1994).

### Conditions which promote release of rAAV particles from producer cells

The present invention provides methods for generating a population of recombinant adeno-associated virus (rAAV) particles, comprising the step of: a) incubating a population of producer cells in a cell culture medium under conditions that promote release of rAAV particles from the producer cells into the culture medium. The released rAAV particles may then be harvested from the cell culture medium, thereby obtaining a population of viral particles.

Conditions which promote (or enhance) release of AAV (including rAAV) particles from a producer cell into the culture medium include, but are not limited to pH of the culture medium; osmolality of the culture medium; temperature; concentration of a given ion in the culture medium; cell density; dissolved oxygen concentration in the culture medium; glucose concentration in the culture medium; concentration of amino acids in the culture medium; and conditions which promote cell cycle synchronization. Any one or more of these parameters is maintained within a suitable range (i.e., a range which promotes release) during which the cells release AAV particles. Although one parameter (i.e., condition) may be sufficient to promote release, a combination of two or more parameters can be simultaneously maintained, each within its own suitable range. Further, a suitable range for one parameter may vary depending on whether any additional parameter(s) used. If one parameter is held within a suitable range for a period of time, a second parameter can be maintained within a second suitable range for the same or a different period of time as the first parameter. Alternatively, conditions may be employed serially. For example, control of pH may occur during one phase of growth, followed by control of temperature.

It is well within the ability of one skilled in the art to vary these parameters and to determine whether release of rAAV into the culture medium is enhanced, relative to the amount of rAAV released when producer cells are not maintained under the given environmental condition(s). Enhanced (or increased) release of rAAV particles from a producer cell can be measured by any of a number of methods known in the art, including, but not limited to, functional assays such as a replication center assay and an infectious center assay; immunological assays for cap gene products, such as ELISA; HPLC; and any of a number of DNA detection methods (to detect the presence of viral DNA), such as slot blot.

Generally, any of these conditions may be monitored and controlled to the extent necessary and/or desired using standard methods and equipment known in the art. The condition is measured and adjustments are made to maintain or return the condition to its suitable level (*i.e.,* a level which promotes virus release). We have observed that failure to appropriately or adequately maintain culture conditions may result in cessation of virus particle release, while other conditions, such as osmolality, need not be maintained at a specific level, but setting of initial culture condition with respect to this parameter(s) is sufficient. For conditions that need to be monitored and adjusted, for example, a bioreactor and/or media perfusion system may be used. These systems are preferred, because they allow more careful control of culture conditions. However, any system which allows sufficient control and adjustment of culture conditions to allow sufficient and/or desired release of AAV particles is suitable. Other examples of control mechanisms for providing the conditions are provided herein.

In one embodiment, producer cells are grown under pH conditions that promote viral particle release. Generally, pH of the culture medium is maintained within a range of about 7.0 to about 8.5, preferably about 7.4 to about 8.5, more preferably about 7.5 to about 8.0. Even more preferably, and especially if pH is the only condition used to promote release, the pH is about 8.0. A bioreactor, for example, permits control of pH to +/- 0.05, and even more precise control is available (such as to +/- 0.01), and can monitor pH every one to 3 minutes or even less. In some embodiments, cells are grown under pH conditions whereby at least about 67% of total virus particles are found in culture supernatant. In other embodiments, cells are grown under pH conditions such that at least about 80%, at least about 82%, at least about 90%, at least about 92%, at least about 95%, of virus particles are found in culture supernatant. Preferably, the P/I, or particle to infectivity ratio, in culture supernatant is less than about 4,000, more preferably less than about 3,000, more preferably less than about 2,000, more preferably less than about 1,700, more preferably less than about 1,500. In some embodiments, cells are cultured at about pH 8 and are harvested on about 96 hours from infection with helper virus (or introduction or intiation of helper virus function(s)). In other embodiments, cells are cultured at about pH 8 and are harvested on about 72 hours from infection with helper virus (or introduction or intiation of helper virus function(s)). In other embodiments, cells are cultured at about pH 8 and are harvested on about 48 hours from infection with helper virus (or introduction or intiation of helper virus function(s)).

In some embodiments a condition or conditions other than pH is used to promote virus release. For example, in other embodiments, temperature is used to promote virus release. Generally, the temperature of the culture medium is maintained between about 30°C to about 45°C, preferably between about 32°C to about 42°C, more preferably about 35°C to about 40°C. Even more preferably, and especially if temperature is the only condition to promote release, the temperature is about 37° to about 39°C. A bioreactor for example can control a temperature to +/- 0.5°C, and can monitor as closely as about every 30 seconds.

In other embodiments, osmolality is used to promote virus release. Generally, the osmolarity of the culture medium is initiated and/or maintained between about 100mOsM to about 650mOsM, preferably about 150mOsM to about 500mOsM, preferably about 200mOsM to about 400mOsM, even more preferably about 300mOsM (especially if osmolarity is the only condition used to promote release). Osmolality, a term well understood in the art, is defined as number of solute molecules per kg water. Generally, compounds such as NaCl and other salts, mannitol, glucose, contribute to osmolality. Osmolality can be measured using standard techniques in the art, such as freezing point depression using, for example, an osmometer.

Other conditions that may be used in the methods of the present invention include, but are not limited to, any one or more of the following: growth factors, such as insulin, EGF and FGF; glucose concentration; dissolved oxygen concentration; enriched media (e.g., additional glucose and/or other nutrients such as vitamins and amino acids). Glucose concentrations are generally between about 0.1 to about 20 g/l; more preferably between about 0.5 to about 15 g/l; more preferably between about 1 to about 10 g/l. Oxygen concentrations (typically measured by, for example, dissolved oxygen electrode or blood gas analyzer) are generally between about 10% to about 200% relative to air, preferably between about 20% to about 100% relative to air, preferably about 30% to about 75% relative to air. Generally, the higher the cell density, the more enriched the media. Media conditions may be maintained and/or supplemented using techniques known in the art, such as perfusion.

Producer cells are grown for a suitable period of time in order to promote release of virus into the media. Generally, cells may be grown for about 24 hours, about 36 hours, about 48 hours, about 72 hours, about 4 days, about 5 days, about 6 days, about 7 days, about 8 days, about 9 days, up to about 10 days. After about 10 days (or sooner, depending on the culture conditions and the particular producer cell used), the level of production generally decreases significantly. Generally, time of culture is measured from the point of viral production. For example, in the case of AAV, viral production generally begins upon supplying helper virus function in an appropriate producer cell as described herein. Generally, cells are harvested about 48 to about 100, preferably about 48 to about 96, , preferably about 72 to about 96, preferably about 68 to about 72 hours after helper virus infection (or after viral production begins). In the Examples, results are usually expressed number of days, e.g., "day 2", "day 3", etc. These designations generally indicate an additional day as measured from infection with helper virus. That is, a result reported for "day 3" generally indicates that the result was obtained approximately 2 days, or 48 hours, from time of introduction of helper virus function(s).

As discussed above, any one or more, in any combination, of the conditions that promote release may be used. For example, cells may be grown under any one or more of the following conditions: (a) pH at about 8.0; (b) temperature of about 39°C; (c) about 300 mOsM; (d) enriched media, for about 2 to 3 days. "Enriched media" generally mean enriched in terms of additional inorganic salts (such as Mg²,Ca⁺²), vitamins, and/or cofactors such that serum may be reduced or even eliminated. In a preferred embodiment, cells are grown under the following conditions: (a) about 300 mOsm; (b) about pH 8.00; (c) about 39°C; (d) and are harvested on about 96 hours from infection with helper virus. In a preferred embodiment, cells are grown under the following conditions: (a) about pH 8.00; (b) about 39°C; and (c) and are harvested on about 96 hours from infection with helper virus. In another embodiment, cells are grown under the following conditions: (a) about 300 mOsm; (b) about pH 8.00; (c) about 39°C; (d) and are harvested on about 72 hours from infection with helper virus. In another embodiment, cells are grown under the following conditions: (a) about pH 8.00; (b) about 39°C; and (c) and are harvested on about 72 hours from infection with helper virus. In another embodiment, cells are grown under the following conditions: (a) about 300 mOsm; (b) about pH 8.00; (c) about 39°C; (d) and are harvested on about 48 hours from infection with helper virus. In another embodiment, cells are grown under the following conditions: (a) about pH 8.00; (b) about 39°C; and (c) and are harvested on about 48 hours from infection with helper virus.

In some embodiments, pH is maintained at about 8.0. and the culture is grown at a temperature of about 39°C. In some embodiments, pH is maintained at about 8.0 and the osmolality (at least the initial osmolality) is about 300 mOsm. In some embodiments, pH is maintained at about 8.0, the osmolality (at least the initial osmolality) is about 300 mOsm, and the culture is grown at a temperature of about 39°C.

In some embodiments, cells are synchronized. This may be accomplished, for example, by subjecting the cells to stress conditions, particularly before addition of helper virus function(s). Synchronization may contribute to overall productivity. Possible forms of stress include, but are not limited to, a nutritional stress, an osmotic stress, a pH stress, a temperature stress, an aerobic stress, a mechanical stress, a radiational stress and a toxic stress. A non-limiting example by which nutritional stress is imposed is by culturing the producer cells in a medium that is deficient in one or more amino acids.

It is also understood that, the invention also includes methods of treating producer cells (i.e., intact producer cells) with an agent or condition that promotes virus release, for example, treatment with an agent which permeabilizes a cell, such as an ionophore or a toxin (such as a bacterial toxin), osmotic shock. For these embodiments, the producer cells of a culture population generally retain their integrity, i.e., are not lysed (although, as in any cell culture population, some cells may be lysed). Generally, less than about any of the following percentage of cells are lysed: 50%, 45%, 40%, 35%, 30%, 25%, 20%, 15%, 12%, 10%, 8%, 5%, 3%, 2%, 1%. Alternatively, generally about at least any of the following percentage of cellular contents are retained in cells (i.e., retained in the cell membrane): 40%, 45%, 50%, 55%, 60%, 65%, 70%, 75%, 80%, 85%, 90%, 95%, 98%.

### Harvesting and Purifying Released Viral Particles

Producer cells may be cultured in suspension or attached to a suitable surface. Methods of suspension or fixed cultures are known in the art. Upon generation of a population of released viral particles, the released viral particles may be harvested and/or purified for further use. As discussed in more detail below, virus particles in culture media are separated from producer cells using methods known in the art, such as centrifugation or filtration (such as tangential flow filtration in a hollow-fiber membrane). Preferably, one or more additional purification steps are performed after separating producer cells from the culture medium. Examples of such steps include, but are not limited to, concentration using suitable filters or ion exchange chromatography. Various production and purification methods are described in WO 99/11764 (PCT/US98/18600) (Targeted Genetics Corporation).

In some embodiments, culture supernatant (after cells are removed) is subjected to opposing ionic chromotography. In some embodiments anion-exchange chromatography is followed by cation-exchange chromotography. In other embodiments, cation-exchange follows anion-exchange chromotography. In some embodiments, culture supernatant is subjected to chromotography on heparin sulphate, preferably after treatment of opposing ionic chromotography, more preferably after treatment of culture supernatant on cation exchange chromotagraphy followed by anion exchange chromatography. These techniques are described in more detailed below.

By way of illustration, culture supernatant, or a preparation which has been eluted from an anion-exchange or cation-exchange column and/or concentrated by tangential flow filtration can be purified by binding to a column comprising heparin sulphate. The AAV can then be eluted from such a column using a buffer containing a salt (eg, a linear gradient of NaCl). For example, AAV obtained from pooled fractions from anion-exchange chromotography column can be concentrated and diafiltered into TMEG plus 100 mM NaCl using a 300K tangential flow filtration membrane. This concentrate may be injected on a one ml heparin sulphate column (Pharmacia "Hi-Trap Heparin" column), and eluted using a linear gradient of NaCl.

The sections below describe in greater detail components of the production system and methods of the invention.

### Producer Cell Comprising Helper Virus Function and AAV

In the methods of the invention, producer cells comprising components necessary for viral replication and encapsidation are cultured under suitable conditions (i.e., conditions that promote viral release). Several criteria influence selection of cells for use in producing rAAV particles as described herein. As an initial matter, the cell must be permissive for replication and packaging of the rAAV vector when using the selected helper virus. However, since most mammalian cells can be productively infected by AAV, and many can also be infected by helper viruses such as adenovirus, it is clear that a large variety of mammalian cells and cell lines effectively satisfy these criteria. Among these, the more preferred cells and cell lines are those that can be easily grown in culture so as to facilitate large-scale production of recombinant AAV vector preparations. Again, however, many such cells effectively satisfy this criterion. Where large-scale production is desired, the choice of production method will also influence the selection of the host cell. For example, as described in more detail below and in the art, some production techniques and culture vessels or chambers are designed for growth of adherent or attached cells, whereas others are designed for growth of cells in suspension. In the latter case, the host cell would thus preferably be adapted or adaptable to growth in suspension. However, even in the case of cells and cell lines that are regarded as adherent or anchorage-dependent, it is possible (as described below) to derive suspension-adapted variants of an anchorage-dependent parental line by serially selecting for cells capable of growth in suspension.

Where a temperature-sensitive helper virus is used, the cell must be able to effectively replicate the rAAV vector under conditions that are non-permissive for replication of the helper virus. By way of illustration, when adenovirus ts149 is used as a ts helper virus (as described below), the cell must be capable of supporting rAAV replication and packaging at temperatures well above 32°C, preferably about 39.5°C. Human 293 cells are an example of a cell line fulfilling these criteria but numerous other cells and cell lines are capable of replicating rAAV at this relatively elevated temperature.

Ultimately, the helper virus (or helper virus function), the rAAV vector sequence, and all AAV sequences needed for replication and packaging must be present in the same cell. Where one or more AAV packaging genes are provided separately from the vector, a host cell is provided that comprises: (i) one or more AAV packaging genes, wherein each said AAV packaging gene encodes an AAV replication or encapsidation protein; (ii) a heterologous polynucleotide introduced into said host cell using an rAAV vector or pro-vector, wherein said rAAV vector or pro-vector comprises said heterologous polynucleotide flanked by at least one AAV ITR and is deficient in said AAV packaging gene(s); and (iii) a helper virus or sequences encoding the requisite helper virus functions. It should be noted, however, that one or more of these elements may be combined on a single replicon. By way of illustration, a helper virus can also comprise an rAAV pro-vector or an AAV packaging gene.

The helper virus is preferably introduced into the cell culture at a level sufficient to infect most of the cells in culture, but can otherwise be kept to a minimum in order to limit the amount of helper virus present in the resulting preparation. A multiplicity of infection or "MOI" of 1-100 may be used, but an MOI of 5-10 is typically adequate.

Similarly, if the AAV vector and/or packaging genes are transiently introduced into the packaging cell (as opposed to being stably introduced), they are preferably introduced at a level sufficient to genetically alter most of the cells in culture. Amounts generally required are of the order of 10 µg per 10⁶ cells, if supplied as a bacterial plasmid; or 10⁸ particles per 10⁵ cells, if supplied as an AAV particle. Determination of an optimal amount is an exercise of routine titration that is within the ordinary skill of the artisan.

These elements can be introduced into the cell, either simultaneously, or sequentially in any order. Where the cell is inheritably altered by any of the elements, the cell can be selected and allowed to proliferate before introducing the next element.

In one preferred embodiment, the helper virus is introduced last into the cell to rescue and package a resident rAAV vector. The cell will generally already be supplemented to the extent necessary with AAV packaging genes. Preferably, either the rAAV vector or the packaging genes, and more preferably both are stably integrated into the cell. It is readily appreciated that other combinations are possible. Such combinations are included within the scope of the invention.

Once the host cell is provided with the requisite elements, the cell is cultured under conditions that are permissive for the replication AAV, to allow replication, packaging and release of the rAAV vector. Suitable culture times depend on a number of considerations and may vary as discussed above. Culture time is preferably adjusted to correspond to peak production levels, and is generally 3-6 days. Preferably, at least 100 viral particles are produced per cell; more preferably at least about 1000 per cell, still more preferably at least about 10,000 per cell. Preferably, at least 0.5 × 10⁶, more preferably at least about 1×10⁶, even more preferably at least about 2×10⁶ RU/ml AAV vectors are produced per 2 × 10⁵ cells during the culture period. Optionally, large-scale production methods such as suspension culture and tangential flow filtration may be used. AAV particles are then collected, and isolated from the cells used to prepare them.

Preparations of rAAV particles obtained by the methods of the present invention preferably comprise a high density of infectious AAV particles and are substantially free of helper virus, helper virus proteins and cellular debris and other contaminants. Desirable properties include the following:
- A concentration of at least 10⁷, preferably at least about 10⁸, more preferably at least about 10⁹ RU/ml, as determined in a replication assay or quantitative hybridization comparison with a known standard.
- No more than 10³, preferably no more than about 10², more preferably no more than about 10¹ infectious particles of helper virus per 10⁸ RU of rAAV particles.
- Less than 5%, preferably less than about 1%, more preferably less than about 0.01%, even more preferably less than about 0.001% contamination by helper virus on a protein basis (wt/wt), detected either by densitometric analysis of SDS gels, or by immunoassay for helper virus specific protein (such as hexon or penton-fiber of adenovirus).
- Less than 5%, preferably less than about 1%, more preferably less than about 0.01 %, even more preferably less than about 0.001 % contamination by helper virus or cellular protein (wt/wt), detected either by densitometric analysis of SDS gels, or by immunoassay for helper virus or cellular specific proteins.
- Preferably, the preparation is also substantially free of other potential cellular components such as cellular lipids, carbohydrates and/or nucleic acids.
   The methods outlined in this disclosure are suitable for preparing small experimental batches, or preparative batches of 10-100 liters or more. For large scale batch preparations, the following property is also desirable:
- A total of at least 10¹⁰, preferably 10¹², and more preferably 10¹⁴ RU of AAV vector particles in the preparation.

Optionally, rAAV vectors may be further processed to enrich for rAAV particles, deplete helper virus particles, or otherwise render them suitable for administration to a subject. Purification techniques may include isopynic gradient centrifugation, and chromatographic techniques. Reduction of infectious helper virus activity may include inactivation by heat treatment or by pH treatment as is known in the art. Other processes may include concentration, filtration, diafiltration, or mixing with a suitable buffer or pharmaceutical excipient. Preparations may be divided into unit dose and multi dose aliquots for distribution, which will retain the essential characteristics of the batch, such as the homogeneity of antigenic and genetic content, and the relative proportion of contaminating helper virus.

Exemplary techniques for generating preparations of helper virus and AAV exhibiting various desirable properties as described above are provided in the following sections.

Various methods for the determination of the infectious titer of a viral preparation are known in the art. One method for titer determination is a high-throughput titering assay, in which an array of culture wells each comprising an aliquot of mammalian cells and an aliquot of virus preparation (as well as control wells comprising e.g., cells alone, virus alone and null) is established. The array of culture wells may, for example, be in the form of a microtiter vessel. Typically, aliquots (e.g., serially diluted aliquots) of the virus preparation to be titered are added to the cells, and then the cells and virus are incubated under conditions that allow for replication of the virus (typically growth conditions suitable for the mammalian host cell). Following replication of the virus, viral nucleic acid is generally released by lysis of the mammalian cells (using conditions or agents that promote lysis as necessary). Nucleic acid (including viral nucleic acid) in the multiplicity of lysates may be transferred and fixed to a membrane under conditions that bind nucleic acid (washing as appropriate to remove proteins and other contaminants). The membrane preferably is a replicate or mirror image of the culture array in which the individual wells of the original array are subsequently represented by "pools" of nucleic acid (from the lysate of each culture well) that are bound at corresponding positions on the membrane. Hybridizing the membrane with a labeled virus-specific (or viral-insert-specific) probe can then be used to identify and quantify the relative amount of viral-specific nucleic acid in each of the points on the array, and by correspondence, in each of the original culture wells. Conditions and materials for nucleic acid transfer, binding, washing and hybridizing can be adapted from routine molecular biological techniques such as "dot blot" hybridization (as described in the art).

### Selection and Preparation of AAV Vector and AAV Packaging Genes

The producer cells used in the production methods described herein comprise an rAAV vector. An rAAV vector comprises a heterologous (i.e. non-AAV) polynucleotide of interest in place of all or a portion of the AAV *rep* and/or *cap* genes that normally make up the bulk of the AAV genome. As in the wild-type AAV genome, however, the rAAV vector is preferably flanked by two AAV inverted terminal repeats (ITRs) as noted above. Variations in which an rAAV construct is flanked by a only a single (typically modified) ITR have also been described in the art and can be employed in connection with the present invention.

Adeno-associated viruses of any serotype are suitable, since the various serotypes are functionally and structurally related, even at the genetic level (see, e.g., Blacklow, pp. 165-174 of "Parvoviruses and Human Disease" J.R. Pattison, ed. (1988); and Rose, Comprehensive Virology 3:1, 1974). All AAV serotypes apparently exhibit similar replication properties mediated by homologous *rep* genes; and all generally bear three related capsid proteins such as those expressed in AAV2. The degree of relatedness is further suggested by heteroduplex analysis which reveals extensive cross-hybridization between serotypes along the length of the genome; and the presence of analogous self-annealing segments at the termini that correspond to ITRs. The similar infectivity patterns also suggest that the replication functions in each serotype are under similar regulatory control. Among the various AAV serotypes, AAV2 is most commonly employed.

An rAAV vector comprises a heterologous polynucleotide. The polynucleotide is typically of interest because of a capacity to provide a function to a target cell in the context of gene therapy, such as up- or down-regulation of the expression of a certain phenotype. Such a heterologous polynucleotide or "transgene", will generally be of sufficient length to provide the desired function or encoding sequence. For encapisdation within AAV2 particles, the transgene will preferably be less than about 5kb although other serotypes and/or modifications may be employed to allow larger sequences to packaged into the AAV viral particles.

Where transcription of the heterologous polynucleotide is desired in the intended target cell, it can be operably linked to its own or to a heterologous promoter, depending for example on the desired level and/or specificity of transcription within the target cell, as is known in the art. Various types of promoters and enhancers are suitable for use in this context. Constitutive promoters provide an ongoing level of gene transcription, while inducible promoters generally exhibit low activity in the absence of the inducer, and are up-regulated in the presence of the inducer. Promoters and enhancers may also be tissue-specific: that is, they exhibit their activity only in certain cell types, presumably due to gene regulatory elements found uniquely in those cells.

Illustrative examples of promoters are the SV40 late promoter from simian virus 40, the Baculovirus polyhedron enhancer/promoter element, Herpes Simplex Virus thymidine kinase (HSV tk), the immediate early promoter from cytomegalovirus (CMV) and various retroviral promoters including LTR elements. Inducible promoters include heavy metal ion inducible promoters (such as the mouse mammary tumor virus (mMTV) promoter or various growth hormone promoters), steroid hormone inducible promoters, and the promoters from T7 phage which are active in the presence of T7 RNA polymerase. Examples of tissue-specific promoters include various surfactin promoters (for expression in the lung), myosin promoters (for expression in muscle), and albumin promoters (for expression in the liver). A large variety of other promoters are known and generally available in the art, and the sequences for many such promoters are available in sequence databases such as the GenBank database.

Where translation is also desired in the intended target cell, the heterologous polynucleotide will preferably also comprise control elements that facilitate translation (such as a ribosome binding site or "RBS" and a polyadenylation signal). Accordingly, the heterologous polynucleotide will generally comprise at least one coding region operatively linked to a suitable promoter, and may also comprise, for example, an operatively linked enhancer, ribosome binding site and poly-A signal. The heterologous polynucleotide may comprise one encoding region, or more than one encoding regions under the control of the same or different promoters. The entire unit, containing a combination of control elements and encoding region, is often referred to as an expression cassette.

The heterologous polynucleotide is integrated by recombinant techniques into or preferably in place of the AAV genomic coding region (i.e. in place of the AAV *rep* and *cap* genes), and is preferably flanked on either side by AAV inverted terminal repeat (ITR) regions. This means that an ITR appears both upstream and downstream from the coding sequence, either in direct juxtaposition, preferably (although not necessarily) without any intervening sequence of AAV origin in order to reduce the likelihood of recombination that might regenerate a replication-competent AAV genome. A single ITR can be sufficient to carry out the functions normally associated with configurations comprising two ITRs (WO 94/13788), and vector constructs with only one ITR can thus be employed in conjunction with the packaging and production methods of the present invention.

The native promoters for *rep* are self-regulating, and can limit the amount of AAV particles produced. The *rep* gene can also be operably linked to a heterologous promoter, whether *rep* is provided as part of the vector construct, or separately. Any heterologous promoter that is not strongly down-regulated by *rep* gene expression is suitable; but inducible promoters are preferred because constitutive expression of the *rep* gene can have a negative impact on the host cell. A large variety of inducible promoters are known in the art; and examples have been listed above. A preferred sub-class of inducible promoters are those that are induced by the helper virus that is used to complement the replication and packaging of the rAAV vector. A number of helper-virus-inducible promoters have also been described, including the adenovirus early gene promoter which is inducible by adenovirus E1A protein; the adenovirus major late promoter; the herpesvirus promoter which is inducible by herpesvirus proteins such as VP 16 or 1CP4; as well as vaccinia or poxvirus inducible promoters.

Methods for identifying and testing helper-virus-inducible promoters, such as promoters derived from the mouse metallothionein gene, have been described See WO96/17947 (Targeted Genetics Corporation).

Given the relative encapsidation size limits of various AAV genomes, insertion of a large heterologous polynucleotide into the genome necessitates removal of a portion of the AAV sequence. Removal of one or more AAV genes is in any case desirable, to reduce the likelihood of generating replication-competent AAV ("rcAAV"). Accordingly, encoding or promoter sequences for *rep, cap,* or both, are preferably removed, since the functions provided by these genes can be provided in *trans.*

The resultant vector is referred to as being "defective" in these functions. In order to replicate and package the vector, the missing functions are complemented with a packaging gene, or a plurality thereof, which together encode the necessary functions for the various missing *rep* and/or *cap* gene products. The packaging genes or gene cassettes are preferably not flanked by AAV ITRs and preferably do not share any substantial homology with the rAAV genome in order to minimize homologous recombination during replication between the vector sequence and separately provided packaging genes. The level of homology and corresponding frequency of recombination increase with increasing length of the homologous sequences and with their level of shared identity. The level of homology that will pose a concern in a given system can be determined theoretically and confirmed experimentally, as is known in the art. Typically, however, recombination can be substantially reduced or eliminated if the overlapping sequence is less than about a 25 nucleotide sequence if it is at least 80% identical over its entire length, or less than about a 50 nucleotide sequence if it is at least 70% identical over its entire length. Of course, even lower levels of homology are preferable since they will further reduce the likelihood of recombination. It appears that, even without any overlapping homology, there is some residual frequency of generating rcAAV. Even further reductions in the frequency of generating rcAAV (e.g. by nonhomologous recombination) can be obtained by "splitting" the replication and encapsidation functions of AAV, as described in WO98/27204 (Targeted Genetics Corporation).

The rAAV vector construct, and the complementary packaging gene constructs can be implemented in this invention in a number of different forms. Viral particles, plasmids, and stably transformed host cells can all be used to introduce such constructs into the packaging cell, either transiently or stably.

The AAV vector and complementary packaging gene(s), if any, may be provided in the form of bacterial plasmids, AAV particles, or any combination thereof. Alternatively, either the AAV vector sequence, the packaging gene(s), or both, are provided in the form of genetically altered (preferably inheritably altered, or stably integrated) eukaryotic cells. The development of host cells inheritably altered to express the AAV vector sequence, AAV packaging genes, or both, provides an established source of the material that is expressed at a reliable level. A variety of different genetically altered cells can thus be used in the context of this invention as described in the art, inter alia, in U.S. Patent 5,658,776; WO95/13392; WO98/23018; U.S. Patent 5,656,785; WO98/27204. Other combinations are possible.

Accordingly, for example, *rep* and *cap* genes may be present in plasmids and/or stably integrated into the genome of the producer cell.

### Introduction of Genetic Material Into Cells

As is described in the art, and illustrated both herein and in the references cited above, genetic material can be introduced into producer cells using any of a variety of means to transform or transduce such cells such as transfection with bacterial plasmids, infection with viral vectors, electroporation, calcium phosphate precipitation, and introduction using any of a variety of lipid-based compositions (a process often referred to as "lipofection"). Methods and compositions for performing these techniques have been described in the art and are widely available.

Selection of suitably altered producer cells may be conducted by any technique in the art. For example, the polynucleotide sequences used to alter the cell may be introduced simultaneously with or operably linked to one or more detectable or selectable markers, such as drug resistance genes, as is known in the art. Testing for acquisition, localization and/or maintenance of an introduced polynucleotide can be performed using DNA hybridization-based techniques (such as Southern blotting and other procedures as known in the art). Testing for expression can be readily performed by Northern analysis of RNA extracted from the genetically altered cells, or by indirect immunofluorescence for the corresponding gene product. Testing and confirmation of packaging capabilities and efficiencies can be obtained by introducing to the cell the remaining functional components of AAV and a helper virus, to test for production of AAV particles. Where a cell is inheritably altered with a plurality of polynucleotide constructs, it is generally more convenient (though not essential) to introduce them to the cell separately, and validate each step seriatim. References describing such techniques include those cited herein.

### Selection and Preparation of Helper Virus

As discussed above, AAV is a parvovirus that is defective for self-replication, and must generally rely on a helper virus to supply certain replicative functions. Various helper viruses can be used in the methods of the present invention.

A number of such helper viruses have been identified, including adenoviruses, herpes viruses (including but not limited to HSV1, cytomegalovirus and HHV-6), and pox viruses (particularly vaccinia). Any such virus may be used with this invention.

Frequently, the helper virus will be an adenovirus of a type and subgroup that can infect the intended host cell. Human adenovirus of subgroup C, particularly serotypes 1, 2, 4, 6, and 7, are commonly used. Serotype 5 is generally preferred. Features and growth patterns of adenovirus are known in the art. See, e.g., to Horowitz, "Adenoviridae and their replication", pp 771-816 in "Fundamental Virology", Fields et al., eds.

Although not essential, in principle it is desirable that the helper virus strain be defective for replication in the subject ultimately to receive the genetic therapy. Thus, any residual helper virus present in an rAAV preparation will be replication-incompetent. Adenoviruses from which the E1A or both the E1A and the E3 region have been removed are not infectious for most human cells. They can be replicated in a permissive cell line (e.g. the human 293 cell line) which is capable of complementing the missing activity. Regions of adenovirus that appear to be associated with helper function, as well as regions that do not, have been identified and described in the art (see, e.g., P. Colosi et al., WO97/17458, and references cited therein).

### Use of a Conditionally-Sensitive Helper Virus

A "conditionally-sensitive" helper virus can also be employed to provide helper virus activity. See WO 99/11764. Such a helper virus strain must minimally have the property of being able to support AAV replication in a host cell under at least one set of conditions where it itself does not undergo efficient genomic replication. Where helper virus activity is supplied as intact virus particles, it is also generally necessary that the virus be capable of replication in a host cell under a second set of conditions. The first set of conditions will differ from the second set of conditions by a readily controllable feature, such as the presence or absence of a required cofactor (such as a cation), the presence or absence of an inhibitory drug, or a shift in an environmental condition such as temperature. Most conveniently, the difference between the two conditions is temperature, and such a conditionally-sensitive virus is thus referred to as a temperature-sensitive helper virus (tsHV).

A "temperature-sensitive" or "ts" helper virus is one which is capable of replicating its genetic material in a eukaryotic cell at a certain temperature range (the "permissive" temperature range), typically about 15°-35°C and preferably about 20-32°C. However, at the "non-permissive" temperature, even when other conditions are kept the same, the rate of replication of genetic material is substantially lower, at least 10-fold lower; usually at least about 100-fold lower; and preferably at least about 1000-fold lower. This temperature is typically about 35°-50°C, generally about 42°C. In a typical example of such a ts helper virus, the virus is capable of efficient replication at relatively low temperatures such as temperatures of about 20-32°C, but is incapable of efficient replication at relatively high temperatures such as temperatures of about 37-42°C. It is understood that the virus-infected cell may nonetheless exhibit some metabolic processes attributable to the virus at the non-permissive temperature, including but not limited to helper function for AAV production.

A temperature-sensitive helper virus can be produced in bulk quantities by culturing infected cells at a permissive temperature. AAV vector can then be produced by culturing cells comprising vector elements and the temperature-sensitive helper virus at a non-permissive temperature. The vector preparation will be substantially free of helper virus components.

A large number of temperature-sensitive adenovirus variants have been described in the art; see, e.g., the variants described by Ensinger et al. (J. Virol. 10:328, 1972); Williams et al. (J. Gen Virol. 11:95, 1971); Ishibashi (Proc. Natl. Acad. Sci. USA 65:304, 1970); Lundholm et al. (Virology 45:827, 1971); and Shiroki et al., (Virology 61:474, 1974). Complementation analysis indicates that such variants fall into a plurality of different complementation groups (Ginsberg et al., Cold Spring Harbor Symp. Quant. Biol. 34:419, 1974). This suggests that a number of steps in the adenovirus replicative cycle may be rendered temperature-sensitive.

Since helper function for AAV replication requires that only part of the adenovirus cycle be intact, testing for helper function of various mutants at the non-permissive temperature provides a means for mapping the helper function. For example, Ishibashi et al. (Virology 45:317, 1971) reported that temperature-sensitive avian adenovirus variants support replication of AAV1 and AAV2. Ito et al. reported that temperature-sensitive mutant ts13 of human adenovirus 7 (Ad7ts13) helps AAV replication at the non-permissive temperature as efficiently as the wild strain. Drake et al. (Virology 60: 230, 1974) reported complementation of AAV4 antigen synthesis by 3 groups of temperature-sensitive mutants of herpes simplex virus type 1 (HSV1). Handa et al. (J. Gen. Viro. 29:239, 1975) reported helper activity for AAV1 virus production by human adenovirus mutants Ad5ts36, Ad5ts125, Ad5ts149, Ad121sA275, Ad12tsB221, and Ad12tsC295. Ostrove et al. (Virology 104:502, 1980) reported that temperature sensitive mutants Ad5ts125, Ad5ts135, Ad5ts157, Ad5ts116, and Ad5ts142, and the host range mutants hr6 but not hr3 support AAV replication. Mayor et al. (J. Gen Virol. 35:545, 1977) reported that Ad31ts13 but not Ad31ts94 supported AAV1 production at the non-permissive temperature.

Straus et al. (Proc. Natl. Acad. Sci. USA 73:742, 1976) reported that Ad5ts125 supported AAV2 replication under conditions where the adenovirus did not itself replicate. They used this property to study DNA intermediates formed during AAV replication. Myers et al. (J. Virol. 35:65, 1980) performed a quantitative study on helper function, and showed that Ad5ts149 supported the production of 20,000 infectious AAV particles per cell at the non-permissive temperature, whereas Ad5ts107 produced only ∼100 particles per cell. Since Ad5ts107 has a mutation in the 72 kDa DNA binding protein encoding region, they concluded that this protein played a role in the AAV RNA expression. More recently, Carter et al. (Virology 191:473, 1992) proposed that a fully functional 72 kDa protein is required for quantitative post-transcriptional expression of the AAV *rep* and *cap* genes.

Condition-sensitive variants of the selected strain of helper virus may be generated by an appropriate mutagenization and selection strategy. For example, virus may be mutagenized with nitrosoguanidine, nitrous acid, hydroxylamine, or 5-bromo-2-deoxyuridine. Candidates are selected that can multiply in a suitable eukaryotic cell under the desired permissive conditions, but not under the desired non-permissive conditions. As an illustration, adenovirus temperature-sensitive mutants can be obtained that multiply, e.g., at 32°C, but not at 39.5°C. Plaquing efficiency ratios at 39.5°C versus 32°C are preferably less than 10⁻⁴ and more preferably less than 10⁻⁵. Further illustration of suitable selection processes for temperature-sensitive adenovirus can be found, for example, in Ensinger et al., J. Virol. 10:328, 1972; and Williams et al., J. Gen Virol. 11:95, 1971. Description of adenovirus variants which are not temperature-sensitive, but host-range sensitive, can be found in Harrison et al., Virology 77:319, 1977. Temperature-sensitive mutants effective for use in this invention can be prepared, for example, from alternative helper viruses like herpes simplex 1 (HSV1), or herpes simplex 2 (HSV2). See, e.g., Schaffer et al., Virology 52:57, 1973 for HSV1; Esparza et al., Virology 57:554, 1974 for HSV2. As indicated in the background section, a large number of condition-sensitive helper viruses have been described, and can be obtained from the scientists who developed or described them or from a public depository.

The strain must be rendered condition-sensitive at a stage in its replicative cycle such that the function that is blocked under non-permissive conditions is not one that is required for high-efficiency replication of AAV. The choice of which helper virus strain to use can be made by reference to both the known biology of the helper virus and the replicative requirements of AAV.

An exemplary helper virus for use with this invention is the temperature-sensitive adenovirus ts149 of the Ad5 serotype (Ad5ts149). Under optimized conditions, this strain can be used to produce rAAV at levels that match or exceed those supported by wild-type Ad5. The ts149 has a single transition of C-G to A-T at position 7563 (Roovers et al., Virus Genes 4:53, 1990). This results in a change of amino acid leucine at residue 411 of the DNA polymerase to phenylalanine. The DNA polymerase is contained within the E2 transcription unit of adenovirus. However, other ts mutants mapping to this region are less suitable. In particular, the E2 transcription unit also comprises the encoding region for the 72 kDa DNA binding protein (DBP). A strain that produces no detectable DBP (Add/802) supports AAV replication, but at a level that is reduced by an order of magnitude (Carter et al., Virology 191:473, 1992). Adts125, which also comprises a mutation mapping to the DBP encoding region, support AAV replication (Straus et al., J. Virol. 17:140, 1976), although the levels are generally much lower than with wild-type Ad5 (Myers et al., J. Virol. 35:65, 1980). Accordingly, suitable temperature-sensitive adenovirus vectors for use in this invention include those for which the sensitivity maps to the E2A region of the genome, preferably to the DNA polymerase encoding region.

The artisan can readily determine which viral strains are suitable for use as helper virus by conducting an rAAV replication assay using a panel of candidate helper virus strains in a candidate cell under conditions that are non-permissive for self-replication of the helper. For temperature-sensitive variants, screening is done at the non-permissive temperature according to the known properties of the strain. Non-permissive temperatures are generally higher than permissive temperatures, typically about 35°-50°C, preferably 38°-45°C, more preferably about 39.5°C. Variants supporting AAV replication at a level that is within one order of magnitude of that supported by the corresponding wild-type virus is preferred. In conducting the screening, the artisan should incorporate the other teachings of this disclosure. In particular, screening by culturing for times that give peak AAV replication with wild-type virus is insufficient. A kinetic matrix should be set up in which the candidate helper viruses are used for longer periods, and then compared with the wild-type virus at peak harvest time.

Once a suitable helper virus strain has been selected, it may be implemented in this invention in a number of different forms. Viral particles, viral plasmids, and stably transformed host cells can all be used.

In one embodiment, the genome of the helper virus (or minimally, the regions of the helper virus genome encoding helper function) is introduced into the host cell to be used for replication of the rAAV vector in the form of a DNA plasmid, or a plurality of plasmids that provide complementary functions. Procedures for experimental manipulation of adenovirus are known in the art. See, for example, Graham et al., "Manipulation of adenovirus vectors". In: Murray EJ, ed Methods in molecular biology: Gene transfer and expression protocols, vol7. Clifton, NJ: The Human Press, 1991:109-128, which provides detailed protocols for propagation, titration, and purification of adenovirus, cotransfection and in vivo recombination. Adenovirus plasmids are available commercially from Microbix Biosystems Inc., Toronto, Canada.

In another embodiment, the host cell is stably transfected with adenovirus genes, or genetically altered to provide the requisite functions for rAAV replication. Alternatively, the host cell may be genetically altered with only a portion of the adenovirus genome, and is subsequently infected or transfected with an adenovirus particle or plasmid. Patent applications WO 95/27071 and WO 95/34671 describe host cells inheritably altered to provide adenovirus function, which complements the replicative property of various defective adenovirus constructs.

In yet another embodiment, the host cell used for AAV replication is infected with a helper virus which is capable of self-replication, but not under non-permissive conditions. Any preparation of the requisite strain providing a sufficient MOI may be used. In keeping with GMP and other regulatory requirements, and to facilitate scale-up for commercial purposes, preparations of helper virus preferably comprise a high density of infectious particles and are substantially free of cellular debris and other contaminants. Desirable properties include the following:
- A density of at least 10⁶, preferably at least about 10⁸, more preferably at least about 10¹⁰ IU/ml, as determined in a TCID₅₀ assay.
- A ratio of adenovirus DNA to total protein or adenovirus hexon that indicates that at least 10%, preferably at least about 50%, more preferably at least about 80% of the viral particles contain adenovirus DNA.
- Less than 20%, preferably less than about 10%, more preferably less than about 1% contamination by non-adenovirus material at the protein or DNA level, as detected by SDS gels stained for protein, or agarose gels of restriction nuclease digests stained with ethidium bromide.
- A total of at least 10⁹, preferably at least about 10¹¹, more preferably at least about 10¹³ IU per production batch.

Helper virus may be prepared in any cell that is permissive for viral replication. For adenovirus, preferred cells include 293 cells and HeLa cells. Traditionally, when these cells have been used for replication of adenovirus, they have been used in plate cultures. These methods generally support replication of temperature-sensitive adenovirus at levels that are one or two logs lower than for wild-type adenovirus.

Accordingly, it is preferable to employ culture techniques that permit an increase in seeding density. 293 cells and HeLa cell variants are available that have been adapted to suspension culture. HeLa is preferable for reasons of cell growth, viability, and morphology in suspension. These cells can be grown at sufficient density (2 × 10⁶ per ml) to make up for the lower replication rate of the temperature-sensitive adenovirus strain. Once established, cells are infected with the virus and cultured at the permissive temperature for a sufficient period; generally 3-7 days and typically about 5 days.

Tangential flow filtration is a technique used in the art for processing large volumes of mammalian cells for the purpose of perfusing, concentrating, and harvesting them. See, e.g., Dorin et al., Biotechnol. Prog. 6:494, 1990; Maiorella et al., Biotechnol. Bioeng. 37:121, 1991. It is recommended that this technique be used with suspension cultures for the preparation of helper virus for use in this invention. HeLa S3 cells withstand shear forces of 750-1500 sec⁻¹, permitting concentration of the cells and diafiltration of spent media.

Virus is harvested from the culture either from the spent media or by microfluidization of the cells. The level of helper virus produced in the culture is typically at least 10⁷ IU/ml, and preferably at least about 3 × 10⁷ IU/ml.

Helper virus prepared according to the foregoing description may be used directly for infecting host cells used for rAAV replication. More usually, the virus is isolated and concentrated before use. Current methods for purifying and concentrating helper virus typically involve isopynic CsCl gradients. This method is time and labor intensive, requires numerous open processing steps, and is difficult to scale up. Instead, purification by chromatography is recommended. The reader is referred generally to Prior et al., Pharmaceut. Technol. 19:30, 1995; and Huyghe et al., Human Gene Therapy 6:1403, 1995. Particularly preferred for isolation of temperature-sensitive strains of adenovirus is anion exchange chromatography, especially on a resin of polyethyleneimine using a continuous NaCl gradient at pH 7.4.

### Preferred AA V Production and Purification Techniques for use in the Present Invention

As with helper virus, it is convenient for purposes of illustration to divide the discussion of AAV production and purification into "upstream" and "downstream" process phases; with the "upstream" process generally referring to the production of AAV in suitable host cells and release of the virus from the cells to produce a "crude" AAV preparation. "Downstream" processing can be employed to purify the AAV preparation (e.g. to isolate AAV away from cellular proteins and/or other contaminants).

In preferred aspects of the present invention, upstream and downstream processing of AAV are conducted in a manner designed to substantially reduce and/or eliminate contaminating cellular proteins, as well as any contaminating helper virus (e.g. Ad) or helper virus proteins, any of which might contribute to elicitation of an immune response if present at substantial levels in the final rAAV vector preparation to be used for gene transfer.

The following sections describe, for purposes of illustration, techniques that can be employed for the production of AAV.

### (i) AAV Upstream Processing

AAV vector can be produced from a mammalian cell line that contains the necessary AAV packaging genes (e.g. an AAV *rep* and *cap* gene); a recombinant AAV (rAAV) pro-vector that comprises a heterologous non-AAV polynucleotide flanked by at least one AAV inverted terminal repeat (ITR); and a helper virus for AAV (e.g. an adenovirus). These components can be introduced into the cell in a variety of configurations. Since AAV can be replicated and packaged in any of a variety of mammalian cells, there are a large number of cell lines that can be modified and employed for the production of AAV.

By way of illustration, AAV vector can be produced from a cell line, such as "C12" (as described by K.R. Clark et al., Gene Therapy, 3: 1124-1132, 1996) or the "Cl37.5" line (described in a commonly-owned copending application by Targeted Genetics Corporation, J. Allen et al., WO 96/17947), that has been engineered to contain a *rep* and/or a *cap* construct, as well as a vector construct. Optionally, a cell line such as C12 or cl37 that contains a *rep* and/or a *cap* construct can be transfected with a plasmid that contains a vector construct, such as ptgAAV-CF. Or a cell can be transfected with a plasmid that contains *rep* and cap, such as pRS5, as well as a plasmid that contains a vector construct. The cell can be infected with Adenovirus, or transfected with DNA that contains adenovirus genes.

A variety of such AAV "producer" cells can be generated, as described in the references cited herein and in the art.

The AAV producer cells can be grown under conditions (including media, temperature and the like) that are generally suitable for growth of the mammalian cells, which are generally also permissive for the replication of AAV. For example, DMEM/F12 suspension medium is preferred for growth of the cells and DMEM medium alone is preferred for AAV vector production. As is known in the art, some cell types and cell lines tend to be attachment-dependent, whereas others are capable of growth in suspension; and many attachment-dependent cells can also be "adapted" to growth in suspension by cycling of the cells under suspension conditions as a means of enriching for and ultimately selecting for variants that are capable of suspension growth. Accordingly, growth of cells for AAV production can be conducted in any of a variety of vessels, depending in part on whether the selected producer cell line is relatively attachment dependent or is suspension adapted. Such vessels for the growth of attachment-dependent cells include, for example, tissue culture flasks, roller bottles, microcarriers and bioreactors (such as hollow-fiber, packed-bed or fluidized-bed bioreactors). Vessels for suspension-adapted mammalian cell lines include, for example, spinner flasks, tank reactors and air lift fermentors.

AAV replication proceeds for a period of time as well as to a point in the growth cycle where viral production is optimal, preferable mid- to late-logarithmic growth (typically one to three days), after which time the virus particles are harvested from the culture supernatant.

Tangential flow filtration (TFF) can be beneficially employed for processing and harvesting large volumes of cells. TFF can be used to perfuse, concentrate and harvest animal cells. For example, TFF can be used to process cells under laminar flow conditions at average wall shear rates of up to 3000 per second (see, e.g., Maiorella, B., et al., Biotechnology and Bioengineering, 37: 121-126, 1991). Large-scale concentration of viruses using TFF ultrafiltration has been described by R. Paul et al. Human Gene Therapy, 4:609-615, 1993.

Illustrative production runs employing such techniques are described below.

### (ii) AAV1 Downstream Processing

As described above, it would be particularly advantageous to obtain preparations of AAV that are substantially free of helper virus particles (such as Ad particles). In addition, AAV vector preparations will preferably also be substantially free of helper virus and cellular proteins (which can also be immunogenic). However, there is a further set of constraints that influence the suitability of techniques for AAV production. Namely, in order to be particularly useful for the production of AAV for gene therapy, it is most desirable for the techniques to be "scalable", i.e. applicable in conjunction with large-scale manufacturing devices and procedures. This latter set of constraints effectively reduces or eliminates the utility of available standard techniques such as cesium chloride separation (which is not well-suited to large-scale preparation procedures).

Preferably, rAAV particles are further purified by ion exchange chromatographic procedures which contrast with various procedures mentioned in the art for the potential purification of, e.g., AAV or Ad. In particular, such procedures as described in the art typically employ a single type of ionic separation, sometimes in combination with other sorts of chromatographic procedures (see, e.g., K. Tamayose et al., Human Gene Therapy 7: 507-513 (1996), and WO96/27677, Sept. 12, 1996). However, in the case of AAV production, a combination of sequential *opposing* ion exchange chromatography is particularly effective for the generation of AAV preparations that are substantially free of helper virus particles and proteins, as well as cellular proteins. A combination of both opposing ionic exchanges is particularly effective for eliminating all of the various particle and protein contaminants that typically occur in the generation or AAV vector preparations.

Any of a variety of cation and anion exchange resins can be employed in conjunction with these procedures, the fundamental properties of which are the availability of negatively- and positively-charged groups, respectively, to which AAV can bind at least to some degree (most preferably to a degree that differs substantially from the relative binding affinity of one or more of the contaminants referred to above, i.e. Ad particles and proteins, as well as mammalian cellular proteins). Without wishing to be bound by theory, it is believed that the anionic exchange step is particularly effective for separating AAV from Adenovirus; whereas both steps (but especially the cationic exchange step) are believed to be particularly effective for separating AAV from cellular proteins. We have also employed anion exchange followed by tangential flow filtration, as described and illustrated below. As further described below, we have found AAV preparations can be highly concentrated by chromatography on heparin sulfate.

By way of illustration, a clarified AAV lysate as described above can be loaded on an positively charged anion-exchange column, such as an N-charged amino or imino resin (e.g. POROS 50 PI, or any DEAE, TMAE, tertiary or quaternary amine, or PEI-based resin) or a negatively charged cation-exchange column (such as HS, SP, CM or any sulfo-, phospho- or carboxy-based cationic resin). The column can be washed with a buffer (such as chromatography buffer A/TMEG). The column can be eluted with a gradient of increasing NaCl concentration and fractions can be collected and assayed for the presence of AAV and/or contaminants.

Other procedures can be used in place of or, preferably, in addition to the above-described anion and cation exchange procedures, based on inter-molecular associations mediated by features other than charge as is known in the art. Such other procedures include intermolecular associations based on ligand-receptor pairs (such as antibody-antigen or lectin-carbohydrate interactions), as well as separations based on other attributes of the molecules, such as molecular sieving chromatography based on size and/or shape. To take just a single example, the filtrate or partially purified AAV preparation may be loaded on a column that contains an AAV-specific antibody. This column can bind AAV. The column can be rinsed with buffer to remove contaminating proteins, and then eluted with a gradient or step of increasing NaCl concentration and fractions can be collected. Alternatively, such a column can be eluted with a buffer of different pH than that of the loading buffer.

The peaks of AAV and adenovirus can be identified in the fractions by infectivity assays or by a nucleic acid hybridization or immunoassays. The peaks can be pooled, and the pool can be diluted or dialyzed or diafiltered with a buffer (e.g. TMEG or equivalent) to reduce the salt concentration.

This pool can be injected on a positively charged anion-exchange column and/or a negatively charged cation-exchange column (such as those referred to above). The column can be washed with a buffer (such as chromatography buffer A/TMEG). The column can be eluted with a gradient of increasing NaCl concentration and fractions can be collected. The peaks of AAV and adenovirus can be identified in the fractions by an infectivity assay or by a nucleic acid hybridization or immunoassay. The peaks can be pooled based on the results of any of these assays.

The pool of AAV-containing fractions eluted from an anion exchange column as described above can be concentrated and purified by tangential flow filtration (TFF), for example in a Filtron Ultrasette or Millipore Pellicon unit. A membrane of suitable molecular weight cut-off (such as a 100,00 or 300,000 cut-off), is typically composed of a polymer such as regenerated cellulose or polyethersulfone. The preparation is filtered through the membrane, and the product is retained. The retained material can be diafiltered using the membrane with successive washes of a suitable buffer such as Ringer's Balanced Salt Solution + 5% glycerol. The final sample is highly enriched for the product and can be sterile filtered through a 0.2µ filter and stored for use.

In the purification and concentration of AAV with tangential flow filtration from post-anionic exchange column material, the 300,000 molecular weight cut-off membrane has resulted in higher yields of replicative units than the 100,000 molecular weight cut-off membrane.

An additional step that can be employed for removal of adenovirus, if desired, involves treating the eluant pool with a heat inactivation step (as described herein) and then filtration (e.g. prior to subjecting the preparation to TFF). However, we have found that the "anion exchange-to-TFF" procedure described above resulted in an AAV preparation that was free of detectable adenovirus, and resulted in better yields of purified AAV.

Illustrative production runs employing such techniques are described below.

The foregoing description provides, inter alia, methods for generating high titer preparations of recombinant AAV vectors that are substantially free of helper virus (e.g. adenovirus) and cellular proteins. It is understood that variations may be applied to these methods by those of skill in this art without departing from the spirit of this invention.

The examples presented below are provided as a further guide to a practitioner of ordinary skill in the art, and are not meant to be limiting in any way.

### EXAMPLES

### EXAMPLE 1

### General methods

### Quantitation of rAAV titers in vector preparations: slot blot assay

The rAAV DNA slot blot assay was conducted as follows. Aliquots of samples were digested with nuclease to remove unencapsidated DNA. The samples were then denatured in 0.4M NaOH, 10 mM EDTA with 1.0 µg/ml salmon sperm DNA at 65°C. Samples and rAAV standards were diluted and filtered onto nylon membranes using a slot blot manifold and washed with 0.4M NaOH. The filter was hybridized with a ³²P-labeled human CFTR cDNA restriction fragment. This probe detects an approximately 1.5 kb fragment from the AAVCF vector (corresponding to the predicted 1.488 kb EcoRI fragment).

### Microtiter infectivity assay to measure rAAV

The microtiter infectivity assay was conducted as previously described. Atkinson et al. (1998) Nucleic Acids Research 26(11): 2821-2823. Briefly, a high-throughput microtiter infectivity assay to measure infectious virus was conducted as follows. Aliquots (10 µl) of serially diluted cell-free supernatants were inoculated onto HeLa clone 37 cells grown in 96-well microtiter plates. After three days, infected cells were treated and lysed with a denaturation solution (addition of 1/10^{th} volume of 4.0 M NaOH, 10 µg/ml salmon sperm DNA and 100 mM EDTA). Lysate was transferred to a Silent Monitor BiodyneB plate (Pall) and vacuum filtered onto the nylon membrane. The membrane was washed, denatured, hybridized with a ³²P-labeled human CFTR cDNA restriction fragment. This probe detects an approximately 1.5 kb fragment from the AAVCF vector (corresponding to the predicted 1.488 kb EcoRI fragment). Vector replication was quantitated relative to an endogenous genomic CFTR band and is expressed as replication units. One replication unit (RU) is defined as a signal intensity equivalent to that of the endogenous genomic CFTR band which is approximately 1.8 kb. Linear regression of serially diluted known vector standards was used to extrapolate and calculate vector concentration in samples.

### Production media

Tables 1 and 2 provide concentrations of components (in mg/l) of media suitable for growing cells and producing rAAV (blanks indicate zero concentration). Generally, it is preferable to have reduced serum levels. For example, the media used in these experiments contained about 1% fetal bovine serum (FBS).

**TABLE 1**

| | **Concentration** |
|---|---|
| **INORGANIC SALTS** | |
| CaCl2 anhydrous | 200 |
| Fe(NO3)3*9H20 | 0.1 |
| KCL | 400 |
| MgSO4*7H20 | 200 |
| NaCl | 4675 |
| NaHC03 | 1200 |
| NaH2PO4•H20 | 125 |

| **OTHER COMPONENTS** | |
|---|---|
| Glucose | 8500 |
| HEPES | 3575 |
| Phenol Red, Na Salt | |
| sodium pyruvate | 110 |
| calcium pantothenate | 6 |
| choline chloride | 6 |
| folic acid | 6 |
| inositol | 11 |
| nicotinamide | 6 |
| pyridoxal HCl | 2 |
| pyridoxine HCl | 4 |
| riboflavin | 0.6 |
| thiamine HCl | 6 |
| F-68 | 500 |

| **AMINO ACIDS** | |
|---|---|
| L- Alanine | 8.9 |
| L-Arginine•HCL | 236.9 |
| L-Asparagine•H20 | 17 |
| L-Aspartic acid | 13.3 |
| L-Cystine | 72 |
| L-Glutamic acid | 14.7 |
| L-Glutamine | 1168 |
| Glycine | 37.5 |
| L-Histidine•HCL•H20 | 84 |
| L-isoleucine | 157.3 |
| L-Leucine | 157.2 |
| L-Lysine•HCL | 218.7 |
| L-Methionine | 45.1 |
| L-Phenylalanine | 99 |
| L-Proline | 11.5 |

| | **Concentration** |
|---|---|
| L-Serine | 52.5 |
| L-Threonine | 142.8 |
| L-Tryptophan | 26.2 |
| L-Tyrosine | 108 |
| L-Valine | 140.4 |
| | |

**TABLE 2**

| *Component* | *Concentration, mg.*/*L* |
|---|---|
| CaCl2 | 200 |
| Fe(NO3)3.9H2O | 0.1 |
| KCI | 400 |
| MgSO4.7H2O | 200 |
| NaCl | 4675 |
| NaHCO3 | 1200 |
| NaH2PO4.H2O | 125 |
| glucose | 4500 |
| HEPES | 3575 |
| sodium pyruvate | 110 |
| calcium pantothenate | 4 |
| choline chloride | 4 |
| folic acid | 4 |
| inositol | 7 |
| nicotinamide | 4 |
| pyridoxal HCl | |
| pyridoxine HCl | 4 |
| riboflavin | 0.4 |
| thiamine HCl | 4 |
| F68 | 500 |
| L-alanine | |
| L-arginine HCl | 84 |
| L-asparagine | |
| L-aspartic acid | |
| L cysteine | 48 |
| Lglutamic | |
| Lglutamine | 876 |
| glycine | 30 |
| I-histidine-HCl. H2O | 42 |
| Lisoleucine | 104.8 |
| L-Leucine | 104.8 |
| L-Lysine HCl | 146.2 |
| L-methionine | 30 |
| L-phenylalanine | 66 |
| L-proline | |
| L-serine | 42 |
| L-threonine | 95.2 |
| L-tryptophan | 16 |
| Ltyrosine | 72 |
| Lvaline | 93.6 |

### EXAMPLE 2

### Effect of pH on rAAV vector particle production in producer cell lysates

JL 14 cells were inoculated at about 3 x 10⁵ cells/ml in a 3 liter bioreactor and grown in 1.5 liters of the rAAV medium shown in Table 2. Two days after inoculating the culture medium in the bioreactor with JL14 cells, the bioreactor was perfused with fresh medium, beginning at 0.4 volumes per day, then doubling this amount every 24 hours thereafter. After 5 days, or when the cell density reached 6 x 10⁶ cells/ml, 3x10⁸ cells were removed and grown under standard conditions, i.e, allowing the pH to vary. The cells remaining in the Bioreactor were concentrated in a volume of 750 ml culture medium, and a three volume medium exchange was performed with production medium (i.e., medium as in Table 2) at pH 7.2 to exchange the medium, so that the final volume of cell culture was 750 ml. Adenovirus type 5 was grown from a stock obtained from the American Type Culture Collection (Manassas, VA). Adenovirus was diluted into 750 ml production medium and added to the cells (multiplicity of infection (MOI) = 10), bringing the final volume to 1.5 liters. Infection with adenovirus was allowed to proceed for one hour at 37°C.

After allowing infection to proceed, 1 x 10⁵ cells were transferred to each of 5 separate spinner flasks. The volume in the five flasks was brought up to 1.5 liters with production medium at pH 6.6, 7.0, 7.2, 7.4, and 7.8, respectively. The contents of the spinner flasks were transferred to separate bioreactors, which were then maintained individually at pH 6.6, 7.0, 7.2, 7.4, and 7.8. Other culture medium parameters were as follows: temperature = 37°C; dissolved oxygen concentration (DO₂) = 30%; and agitation = 150 rpm.
Temperature, pH, DO₂, cell density, osmolarity and glucose/lactate were monitored daily. Cell samples were harvested on day 2 and day 3 post-infection and lysed. The cell lysates were assayed by DNAse-resistant particles (DRP) slot blot assay and by the microtiter infectivity assay.

Figures 1A and 1B are bar graphs depicting the results of two separate experiments, expressed as DRP per cell at the various pH levels. Solid bars represent DRP/cell at day 2 post-infection; hatched bars represent the DRP/cell at day 3. The DRP/cell in the cultures maintained at pH 7.2, pH 7.4, and pH 7.8 decreased dramatically from day 2 to day 3 post-infection, while this reduction was not as pronounced in the control cell culture. The total cell density did not change appreciably under these culture conditions.

### EXAMPLE 3

### Effect of pH on release of rAAV vector particles into the cell culture medium

Briefly, AAV producer cells were grown in bioreactors. Cells were then infected with Ad5 at MOI=10 and inoculated into low-serum media (see Table 2) in suspension in 1.5 liter bioreactors. Cultures were maintained at various elevated pH levels (from 7.2 to 8.0). Cultures were then monitored daily for cell number, viability, glucose consumption, lactate production, pH, osmolarity and AAV production. As shown below, there was an increase in AAV production when the pH was elevated to 7.4; coupled with an even more dramatic increase in the number of AAV particles released into the supernatant (which increased as pH was elevated):

| Culture pH | Cell-associated Particles | Supernatant Particles | Total Particles | % Cell-associated | % in Supernatant |
|---|---|---|---|---|---|
| 7.2 | 4.70E + 12 | 1.90E + 09 | 4.70E + 12 | 100% | 0% |
| 7.4 | 6.50E + 12 | 1.30E + 13 | 1.95E + 13 | 33% | 67% |
| 7.6 | 3.40E + 12 | 1.50E + 13 | 1.84E + 13 | 18% | 82% |
| 8.0 | 1.30E + 12 | 1.50E + 13 | 1.63E + 13 | 8% | 92% |

In sum, as pH was raised, we observed a sharp increase in the number of AAV particles released into the supernatant, and a shift in the percentage of supernatant:cell-associated particles (from nearly all cell-associated at pH 7.2 to mostly supernatant (92%) at pH 8.0).

To further investigate the effects of pH on rAAV vector production, JL14 cells were grown in a perfusion bioreactor as described above (using media described in Table 1) to a density of 10⁷ cells/ml. The cell culture was concentrated to a volume of 750 ml and the medium exchanged by performing 3 diavolumes. The total volume was brought to 1.5 liters with production medium containing adenovirus at a MOI of 10. Infection was allowed to proceed for one hour.

After allowing infection to proceed, 1 x 10⁵ cells were transferred to each of 5 separate spinner flasks. The volume in the five flasks was brought up to 1.5 liters with production medium at pH 7.2, 7.4, 7.6, 7.8, 8.0, and control flasks (pH not maintained at the starting level). The contents of the spinner flasks were transferred to separate bioreactors, which were then maintained individually at pH 7.2, 7.4, 7.6, 7.8, 8.0. Bioreactors maintained the pH at the stated level ± 0.05 pH units. Other culture medium parameters were as follows: temperature = 37°C; dissolved oxygen concentration (DO₂) = 30%; and agitation = 150 rpm. Cells and culture supernatants (culture media) were harvested on days 2 and 3.

The results are shown in Figures 2A and 2B. Figures 2A and 2B are bar graphs and depict the results, expressed as total DRPs, of rAAV production in bioreactors maintained at various pH levels. Percentages above each bar are percentages of total DRPs in the cell lysate. The solid portion of each bar represents DRPs in cell lysates, while the hatched portion of each bar represents the DRPs in the cell culture medium. On day 2 post-infection, 29% of the total DRPs were in the culture medium of the culture maintained at pH 8.0, while on day 3, post-infection, the percent of total DRPs in the culture medium rose to 92%. On day 3 post-infection, the percentage of total DRPs in the culture medium was 67% at pH 7.4, 82% at pH 7.6, 73% at pH 7.8 and 92% at pH 8.0. Cultures maintained at pH 7.2 did not yield any DRPs in the cell culture medium in this experiment.

The day 2 and day 3 post-infection cell lysates and culture media from the bioreactors maintained at pH 7.2, 7.4, 7.6, 7.8, and 8.0 were assayed for replication units (RU) using an infectivity assay. The data are shown in Figures 3A and 3B. The total cell density did not change appreciably under these culture conditions.

Figures 3A and 3B are bar graphs depicting the total replication units (RU) assayed at day 2 (3A) and day 3 (3B) post-infection in the culture media (hatched portion of each bar) and cell lysates (solid portion of each bar) when cultures were maintained at the indicated pH levels. Percentages above each bar indicate the percentage of total RUs in the cell lysate. These data demonstrate that the rAAV particles released into the cell culture medium are functional in an infectivity assay.

Figure 4 is a bar graph depicting the particle:infectivity (P/I) ratio of rAAV particles harvested from cell lysates (solid portion of each bar) and cell culture medium (hatched portion of each bar) at day 3 post-infection from bioreactors maintained at the indicated pH levels. These date indicate that the majority of the rAAV vector released into the cell culture medium is infectious.

### EXAMPLE 4

### Effect of osmolality on release of rAAV vector particles into the cell culture medium

To assess the effects on release of rAAV into the cell culture medium of starting osmolality of the culture medium, JL14 cells were grown in bioreactors and infected with adenovirus essentially as described in Example 2. The starting osmolality in the individual bioreactors was 130, 200, 300, 400, and 500 mOsm, respectively. In each reactor, the pH was maintained at pH 8.0 (±0.05); temperature = 37°C; DO₂ = 30%; and agitation = 150 rpm. On days 2, 3, and 4 post-infection, cell lysates and cell culture media were collected and analyzed for rAAV vector production.

The results are shown in Figures 5-7.

Figures 5A, 5B, and 5C are bar graphs depicting the total DRPs in cell lysates (solid portion of each bar) and cell culture media (hatched portion of each bar) on day 2 (5A), day 3 (5B), and day 4 (5C) post-infection in bioreactors in which the cell culture media contained the indicated starting osmolality. Percentages above each bar indicate the percentage of total DRPs in the cell lysate. These data show that when the cell culture medium has a starting osmolality of 300 mOsm, 41%, 59%, and 80% of the total DRPs are in the cell culture medium at day 2, 3, and 4, respectively. A starting cell culture medium osmolality of 300 mOsm gave the maximum percentage of total rAAV vector in the cell culture medium, compared with other starting osmolalities tested. The total cell density did not change appreciably under these culture conditions.

Figures 6A, 6B, and 6C are bar graphs depicting the total RUs in cell lysates (solid portion of each bar) and cell culture media (hatched portion of each bar) on day 2 (6A), day 3 (6B), and day 4 (6C) post-infection in bioreactors in which the cell culture media contained the indicated starting osmolality. Percentages above each bar indicate the percentage of total RUs in the cell lysates. These data indicate that the rAAV vector released into the medium is infectious.

Figure 7 is a bar graph depicting the P/I ratio of rAAV particles in cell culture media at days 3 and 4 from bioreactor cultures with the indicated starting osmolalities.

### EXAMPLE 5

### Effect of temperature on release of rAAV vector particles into the cell culture medium

JL 14 cells were grown in bioreactors and infected with adenovirus essentially as described in Example 2. Cells were transferred to bioreactors maintained individually at 31°C, 34°C, 37°C, 39°C, and 42°C, respectively. These temperatures were maintained ± 0.5 °C. The pH in each reactor was maintained at 8.0 (±0.05); agitation = 150 rpm; DO2 = 30%. On days 2, 3, and 4 post-infection, cell lysates and cell culture media was analyzed for rAAV particles.

The results are shown in Figures 8 and 9.

Figures 8A-C are bar graphs depicting the total DRPs in cell lysates (solid portion of each bar) and cell culture media (hatched portion of each bar) on day 2 (8A), day 3 (8B), and day 4 (8C) post-infection in bioreactors in which the cell culture media was maintained at the indicated temperature. Percentages above each bar indicate the percentage of total DRPs in the cell lysate. The data show that when the culture medium was maintained at 39°C, 66%, 67%, and 57% of the total DRPs were found in the cell culture medium on days 2, 3, and 4, respectively. The data further indicate that a higher percentage of DRPs was found in the cell culture medium when the culture media was maintained at 39°C, compared to 37°C or 42°C.

Figures 9A, 9B, and 9C are bar graphs depicting the total RUs in cell lysates (solid portion of each bar) and cell culture media (hatched portion of each bar) on day 2 (9A), day 3 (9B), and day 4 (9C) post-infection in bioreactors in which the cell culture media was maintained at the indicated temperature. Percentages above each bar in Figure 9A indicate the percentage of total RUs in the cell culture medium. Percentages above each bar in Figure 9A indicate the percentage of total RUs in the cell lysate. These data show that when the culture medium was maintained at 39°C, 80%, 97%, and 98% of total RUs were found in the cell culture medium on days 2, 3, and 4, respectively. The total cell density did not change appreciably under these culture conditions.

### EXAMPLE 6

### Effect of culture medium supplements on release of rAAV vector particles into the cell culture medium

JL14 cells were grown in bioreactors and infected with adenovirus essentially as described in Example 2. Cells were transferred to bioreactors containing various media, as follows: (1) DMEM; (2) DMEM + 4 g/liter glucose; (3) DMEM + 4 g/liter glucose + 4 mM glutamine; (4) DMEM + 4 g/liter glucose + 4 mM glutamine + amino acids + vitamins ("complete"); (5) 2X DMEM. All starting osmolalities were adjusted to 285-300 mOsm. Other parameters were as follows: temperature maintained at 39°C; pH maintained at 8.0; DO₂ = 30%; and agitation = 150 rpm. Three days post-infection, the cell culture supernatant was assayed for rAAV vector particles.

The results are shown in Figures 10, 11, and 12.

Figure 10 is a bar graph depicting the total DRPs in the culture media three days post-infection in cultures grown in the various media indicated.

Figure 11 is a bar graph depicting the RUs in the culture media three days post-infection in cultures grown in the various media indicated.

Figure 12 is a bar graph depicting the P/I ratio of viral particles in the cell culture media when cultures were grown in the various media indicated.

Although the foregoing invention has been described in some detail by way of illustration and example for purposes of clarity of understanding, it will be apparent to those skilled in the art that certain changes and modifications will be practiced. Therefore, the description and examples should not be construed as limiting the scope of the invention, which is delineated by the appended claims.

### Preferred embodiments of the present invention are described below and referred to as embodiments E1 to E25

E 1. A method of generating a population of virus particles, comprising the step of:
   a) incubating a producer cell in a cell culture medium under conditions comprising a condition that promote release of virus particles, whereby virus particles are released from the producer cell into the culture medium.
E 2. The method of E1, wherein the virus is recombinant adeno-associated virus (rAAV), and wherein the producer cell comprises:
   (i) one or more AAV packaging genes, wherein each said AAV packaging gene encodes an AAV replication or encapsidation protein;
   (ii) a recombinant AAV (rAAV) vector that comprises a heterologous non-AAV polynucleotide flanked by at least one AAV inverted terminal repeat (ITR); and
   (iii) helper virus function for AAV.
E3. The method of E 2, wherein the condition that promotes release of virus particles is pH.
E 4. The method of E 3, wherein the pH is about 7.4 to about 8.0.
E 5. The method of E 4, wherein the pH is about 8.0.
E 6. The method of E 2, wherein the condition that promotes virus release is osmolality.
E 7. The method of E 6, wherein the osmolality is about 300 mOsm.
E 8. The method of E 5, wherein the initial osmolality of the cell culture is about 300 mOsm.
E9. The method of E 2, wherein the condition that promotes virus release is temperature.
E 10. The method of E 9, wherein the temperature is about 37°C to about 40°C.
E11. The method of E 10, wherein the temperature is about 39°C.
E12. The method of E 5, wherein the temperature is about 39°C.
E 13. The method of E 8, wherein the temperature is about 39°C.
E 14. The method of E5, wherein producer cells are cultured for about 48 to about 96 hours after introduction of helper virus function.
E15. The method of E 12, wherein producer cells are cultured for about 48 to about 96 hours after introduction of helper virus function.
E 16. The method of E 2, wherein helper virus function is provided by helper virus.
E17. A method of generating a population of rAAV particles according to E 16, wherein said helper virus is an adenovirus.
E 18. The method of E 2, further comprising the step of (b) harvesting the viral particles from the cell culture medium, thereby obtaining a population of rAAV particles.
E19. The method of E18, further comprising the steps of:
   c) chromatographing the AAV producer cell culture medium on at least one positively-charged anion exchange resin; and
   d) purifying the chromatographic fractions containing rAAV particles of step c) by cation exchange chromatography or tangential flow filtration to generate a purified population of rAAV vector particles.
E 20. A method of generating a population of rAAV particles according to E2, wherein said rAAV vector comprises a heterologous non-AAV polynucleotide flanked by two AAV inverted terminal repeats (ITRs).
E21. A method of generating a population of rAAV particles according to E 2, wherein said AAV producer cell comprises at least one AAV packaging gene that is stably integrated into the genome of said AAV producer cell.
E22. A method of generating a population of rAAV particles according to E 2, wherein said AAV producer cell comprises an AAV *rep* gene and an AAV *cap* gene.
E23. A method of generating a population of rAAV particles according to E22, wherein said AAV *rep* gene and AAV *cap* gene are stably integrated into the genome of said AAV producer cell.
E 24. A method of generating a population of rAAV particles according to E 2, wherein the producer cell is an attachment-dependent mammalian cell line.
E 25. A method of generating a population of rAAV particles according to E 2, wherein the producer cell is a suspension-adapted mammalian cell line.

## Claims

1. A method of generating a population of virus particles, comprising the step of:
a) incubating a produce cell in a cell culture medium under conditions comprising a condition that promote release of virus particles, whereby virus particles are released from the producer cell into the culture medium without lysing the cells, wherein the condition that promotes release of virus particles comprises one or more conditions selected from osmolality, temperature, concertation of a given ion, cell density, dissolved oxygen concentration, glucose concentration, amino acid concentration and a condition with promotes cell cycle synchronization; and
b) harvesting the viral particles from the cell culture medium without lysing the cells, thereby obtaining a population of viral particles;
wherein the virus is a non-lytic and/or non-budding virus.

2. The method of claim 1, wherein the virus is recombinant adeno-associated virus (rAAV), and wherein the producer cell comprises:
(i) one or more AAV packaging genes, wherein each said AAV packaging gene encodes an AAV packaging replication or encapsulation protein;
(ii) a recombinant AAV (rAAV) vector that comprises a heterologous non-AAV polynucleotide flanked by at least one AAV inverted terminal repeat (ITR); and
(iii) helper virus function for AAV.

3. The method of claim 1 or 2, wherein the condition that promotes release of virus is osmolality, temperature or a combination of osmolality and temperature.

4. The method of claim 3, wherein the osmolality is maintained at 200 mOsm to 400 mOsm, preferably wherein the osmolality is 300 mOsm.

5. The method of any one of claims 3 to 5, wherein the osmolality of the cell culture is adjusted using NaCl or another salt, mannose or glucose, preferably adjusted using NaCl.

6. The method of claim 3, wherein the temperature is 37°C to about 40°C, preferably wherein the temperature is 39°C.

7. The method of claim 1 or 2, wherein the condition that promotes virus release is cell density, glucose concentration and/or amino acid concentration.

8. The method of any one of the preceding claims, wherein producer cells are cultured for 48 to 96 hours after introduction of helper virus function.

9. The method of claim 2, wherein helper virus function is provided by helper virus or by a polynucleotide sequence of said helper virus that encodes at least one helper virus function, preferably, wherein said helper virus is an adenovirus, herpesvirus or poxvirus, optionally wherein said herpesvirus is herpes simplex virus, Epstein-Barr virus, cytomegalovirus or pseudorabies virus.

10. The method of any of claims 2 to 9, further comprising the steps of:
c) chromatographing the AAV producer cell culture medium on at least one positively-charged anion exchange chromatography; and
d) purifying the chromatographic fractions containing rAAV particles of step c) by cation exchange chromatography or tangential flow filtration to generate a purified population of rAAV vector particles.

11. The method of any one of claims 2 to 9, further comprising the steps of:
c) chromatographing the AAV producer cell culture medium on a cation exchange chromatography; and
d) purifying the chromatographic fractions containing rAAV particles of step c) by anion exchange chromatography; and
e) purifying the chromatographic fractions containing rAAV particles of step d) by heparin sulphate chromatography to generate a purified population of rAAV vector particles.

12. The method of any one of claims 2 to 9, further comprising the steps of chromatographing the AAV producer cell culture medium on a plurality of ion-exchange chromatographies comprising at least one positively-charged anion exchange chromatography and at least one negatively-charged cation exchange chromatography to generate a purified population of rAAV vector particles.

13. A method of generating a population of rAAV particles according to any one of claims 2 to 12, wherein said rAAV vector comprises a heterologous non-AAV polynucleotide flanked by two AAV inverted terminal repeats (ITRs).

14. A method of generating a population of rAAV particles according to any one of claims 2 to 13, wherein said AAV producer cell comprises at least on AAV packaging gene that is stably integrated into the genome of said AAV producer cell.

15. A method of generating a population of rAAV particles according to any one of claims 2 to 12, wherein said AAV producer cell comprises an AAV *rep* gene and an AAV *cap* gene, preferably wherein said AAV *rep* gene and AAV *cap* gene are stably integrated into the genome of said AAV producer cell.

16. A method of generating a population of rAAV particles according to any one of claims 2 to 12, wherein the producer cell is an attachment-dependent mammalian cell line or a suspension-adapted mammalian cell line.
